(19) Europäisches Patentamt
European Patent Office
Office européen des brevets

(11) Numéro de publication : **0 457 686 B1**

# (12) FASCICULE DE BREVET EUROPEEN

(45) Date de publication du fascicule du brevet : **03.08.94 Bulletin 94/31**

(21) Numéro de dépôt : **91401274.5**

(22) Date de dépôt : **17.05.91**

(51) Int. Cl.$^5$ : **C07D 211/58,** C07D 211/26, C07D 207/09, C07D 207/14, C07D 405/06, C07D 471/04, A61K 31/445

(54) **Nouveaux dérivés de l'amino pipéridine de l'amino pyrrolidine et de l'amino perhydroazépine, leurs procédés de préparation et les compositions pharmaceutiques qui les contiennent.**

(30) Priorité : **18.05.90 FR 9006220**

(43) Date de publication de la demande : **21.11.91 Bulletin 91/47**

(45) Mention de la délivrance du brevet : **03.08.94 Bulletin 94/31**

(84) Etats contractants désignés : **AT BE CH DE DK ES FR GB GR IT LI LU NL SE**

(56) Documents cités :
**EP-A- 0 035 374**
**EP-A- 0 160 422**
**EP-A- 0 277 794**
**US-A- 3 910 931**

(73) Titulaire : **ADIR ET COMPAGNIE**
**1 rue Carle Hébert**
**F-92415 Courbevoie Cédex (FR)**

(72) Inventeur : **Peglion, Jean-Louis**
**5 Allée des Bégonias**
**F-78110 Le Vesinet (FR)**
Inventeur : **Colpaert, Francis**
**36 bis Boulevard Carnot**
**F-78110 Le Vesinet (FR)**

(74) Mandataire : **Reverbori, Marcelle**
**Adir et Compagnie,**
**1, rue Carle Hébert**
**F-92415 Courbevoie Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 457 686 B1

Jouve, 18, rue Saint-Denis, 75001 PARIS

## Description

La présente invention a pour objet des nouveaux dérivés de l'amino pipéridine, de l'amino pyrrolidine et de l'amino perhydroazépine, leurs procédés de préparation et les compositions pharmaceutiques qui les contiennent.

Certains dérivés de la 4-benzoylamino 1-cycloalcenylméthyl pipéridine ayant des activités antagonistes de la dopamine et stimulantes des récepteurs de la sérotonine sont décrits dans la demande de brevet FR-2369.263. Les demandes EP-160.422, EP-256.798 et EP-277.794 décrivent des N-aryl N-(4-pipéridyl) amides ayant des propriétés analgésiques, anesthésiques et sédatives.

La demande FR-2370.731 décrit des amides, dérivés de la pipéridine ayant l'aptitude à neutraliser les effets de la dopamine ou des agents dopaminergiques.

Certains amides, dérivés de la pyridine, tétrahydropyridine et pipéridine ayant des activités hypotensives, anti-inflammatoires et analgésiques sont décrits dans le brevet GB-1410783, et certains N-aryl N-pipéridyl arylacétamides ayant des propriétés antiarrythmiques sont décrits dans la demande de brevet FR-2325.377

L'état antérieur de la technique est également illustré par les brevets : EP-A-0035 374, US-A-3 910 931, EP-A 0160 422 et EP-A-02777 94, parmi lesquels le document le plus proche de la présente demande est la demande de brevet EP-A-0 035 374.

Toutefois, les dérivés de la présente invention se distinguent de ceux de la technique antérieure par leurs structures originales et leurs propriétés pharmacologiques. Les essais pharmacologiques ont démontré que les composés de l'invention sont des antagonistes des récepteurs $5\text{-}HT_{1A}$. Certains d'entres eux présentent également une bonne affinité pour le récepteur sigma. Les dérivés de l'invention peuvent donc être utiles dans le traitement de la douleur, du stress, de la migraine, de l'anxiété, de la dépression et de la schizophrénie.

La présente invention a plus particulièrement pour objet les dérivés de formule I :

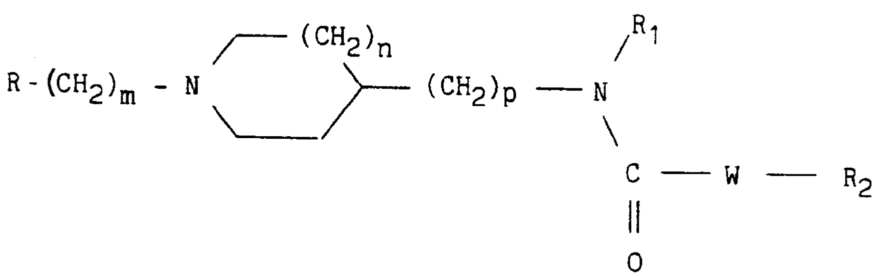

(I)

dans laquelle :

- m représente zéro, 1, 2, 3 ou 4,
- n et p représentent zéro, 1 ou 2,
- W représente un atome d'oxygène , un radical -NH-, ou une simple liaison,
- R représente

  un radical benzocyclobutén-l-yle de formule B :

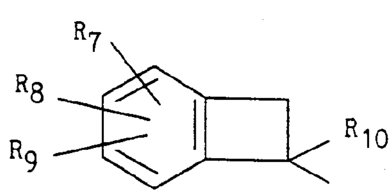

(B)

  un radical indanyle de formule C

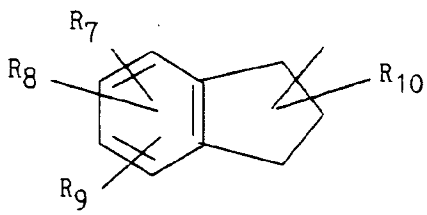

(C)

(dans lesquelles :

- $R_7$, $R_8$ et $R_9$ identiques ou différents représentent chacun un atome d'hydrogène, un atome d'halogène, un radical alkyle ou alcoxy de 1 à 6 atomes de carbone, un radical alkyle polyhalogéné de 1 à 6 atomes de carbone, un radical hydroxy, ou $R_7$ et $R_8$ ou $R_8$ et $R_9$ forment ensemble un radical méthylènedioxy, un radical éthylènedioxy, un cycle furanique ou dihydrofuranique et
- $R_{10}$ représente un atome d'hydrogène ou un radical alkyle de 1 à 6 atomes de carbone ;

- $R_1$ représente un radical alkyle de 1 à 6 atomes de carbone,
- $R_2$ représente un atome d'hydrogène, un radical alkyle de 1 à 6 atomes de carbone, un radical alcène de 2 à 6 atomes de carbone, un radical cycloalkyle de 4 à 7 atomes de carbone, un radical benzyle ou un radical phényle (chacun- éventuellement substitué par un ou plusieurs atomes d'halogènes, radicaux hydroxy, ou des radicaux alkyle ou alkoxy de 1 à 6 atomes de carbone), un radical aralkyle de 7 à 12 atomes de carbone, un radical alcoxyalkyle de 2 à 7 atomes de carbone ou un radical alkyle polyhalogéné de 1 à 6 atomes de carbone,

leurs isomères optiques et leurs sels d'addition avec un acide organique ou minéral pharmaceutiquement acceptable.

La présente invention a également pour objet un procédé de préparation des composés de formule générale I, caractérisé en ce que :

a) soit on condense :
un composé de formule II

$$R\text{-}(CH_2)_m\text{-}X \qquad (II)$$

dans laquelle R et m ont la même signification que pour la formule I, et X représente un atome d'halogène, un radical tosyloxy ou un radical mésyloxy, avec un composé de formule III :

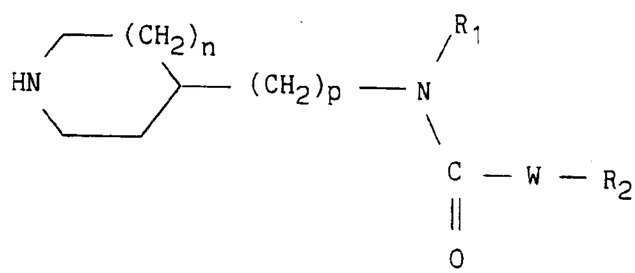

(III)

dans laquelle n, p, $R_1$, W et $R_2$ ont la même signification que pour la formule I,
pour former les composés de formule I,

b) soit on fait réagir un composé de formule IV :

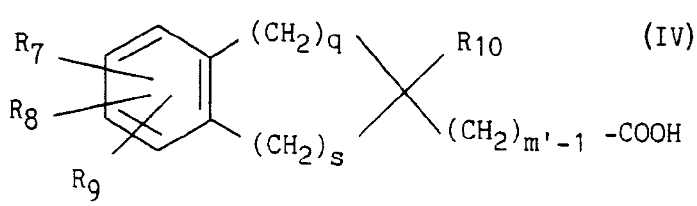

(IV)

dans laquelle $R_7$, $R_8$, $R_9$ et $R_{10}$ ont les significations précédemment définies, m' représente 1,2,3 ou 4 et q et s représentent 0, 1 ou 2 à condition toutefois que q + s soit égal à 1 ou 2,
avec un composé de formule V :

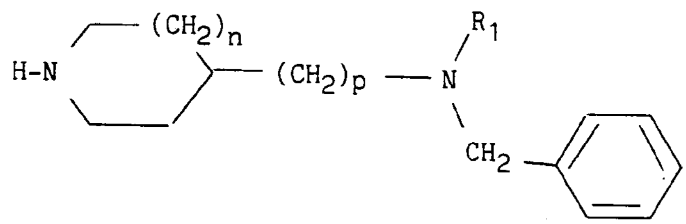

(V)

dans laquelle n, p et $R_1$ ont la même signification que pour la formule I, pour former un composé de formule VI :

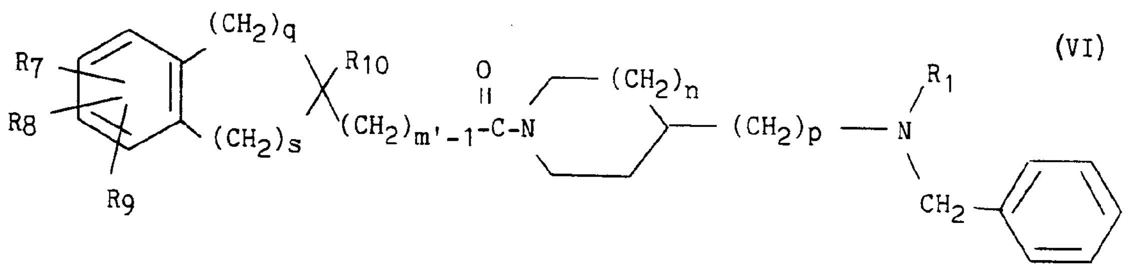

(VI)

dans laquelle $R_1$, $R_7$, $R_8$, $R_9$, $R_{10}$, n, p, q et s ont les significations indiquées ci-dessus, et m' représente 1,2,3 ou 4,
que l'on soumet à l'action de l'hydrure de lithium et d'aluminium pour former un composé de formule VII :

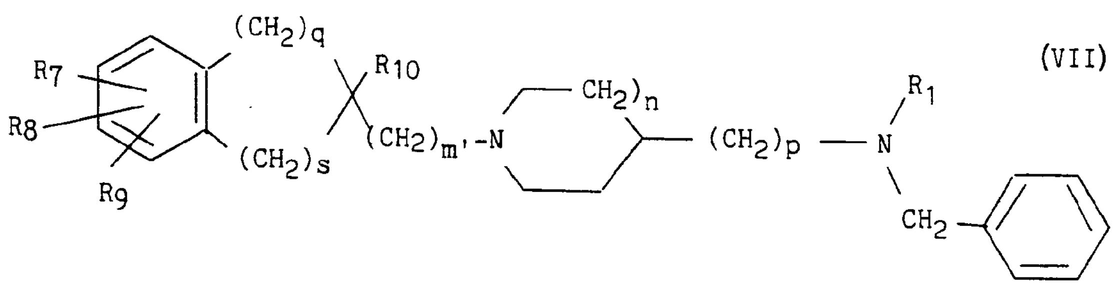

(VII)

dans laquelle les significations de $R_1$, $R_7$, $R_8$, $R_9$, $R_{10}$, m', n p, q et s restent identiques à celles données précédemment,

que l'on soumet à l'action d'hydrogène, en présence d'un catalyseur tel que $FtO_2$ ou Pd/C, dans un solvant alcoolique, en présence d'une quantité stoechiométrique d'acide chlorhydrique ou acétique, pour former un composé de formule VIII

(VIII)

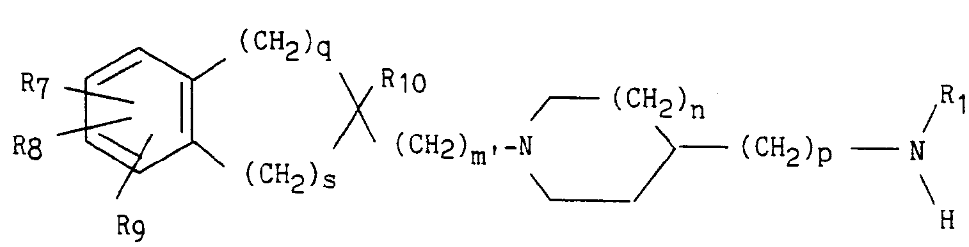

dans laquelle $R_1$, $R_7$, $R_8$, $R_9$, $R_{10}$, m', n, p, q et s ont les significations indiquées ci-dessus,

lequel est ensuite

α) soit mis à réagir avec un composé de formule IX :

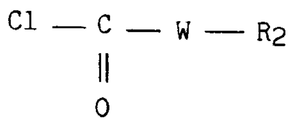

(IX)

dans laquelle la signification de $R_2$ est identique à celle donnée pour la formule I mais il ne représente pas un atome d'hydrogène, et W représente une simple liaison ou un atome d'oxygène,

pour former les composés de formule I, dans laquelle R représente un radical B ou un radical C, W représente une simple liaison ou un atome d'oxygène, $R_2$ a la signification précédemment définie mais ne représente pas un atome d'hydrogène, et m a la signification précédemment définie à l'exception de zéro,

β) soit mis à réagir avec l'acide formique en présence d'anhydride acétique pour obtenir les composés de formule I dans laquelle R représente un radical B ou un radical C, W représente une simple liaison, $R_2$ un atome d'hydrogène, et m a la signification précédemment définie à l'exception de zéro,

γ) soit mis à réagir avec un composé de formule X

$$R_2 - N = C = O \qquad (X)$$

dans laquelle $R_2$ a la même signification que pour la formule I à l'exception d'un atome d'hydrogène, pour former les composés de formule I dans laquelle R représente un radical B ou un radical C, W représente un radical -NH- , $R_2$ a la signification précédemment définie à l'exception d'un atome d'hydrogène, et m a la signification précédemment définie à l'exception de zéro,

lesquels composés de formule I, sont ensuite, si on le désire :

- salifiés avec un acide minéral ou organique pharmaceutiquement acceptable,

  ou

- séparés en leurs isomères optiques et ensuite salifiés.

Les composés de formule II, quand R représente un radical (B) sont obtenus à partir des acides de formule XIII :

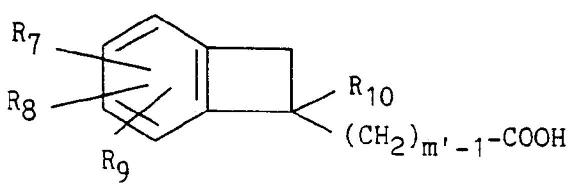

(XIII)

dans laquelle $R_7$, $R_8$, $R_9$ et $R_{10}$ ont les significations précédemment définies, et m' représente 1,2,3 ou 4, selon un procédé déjà décrit dans la littérature (J.A.C.S., (1975), 154, p.347). Les procédés de synthèse des acides de formule XIII ou de leurs dérivés sont aussi connus.(J.A.C.S., (1958), 80, p.2257 ; J.A.C.S., (1975), 157, p.347 ; J. Chem., (1972), 32, p.820 ; J. Org. Chem., (1968), 33, p.3327 ; Tet. Lett.,(1973), 29, p.73).

Les dérivés de la pipéridine de formule $III_A$ :

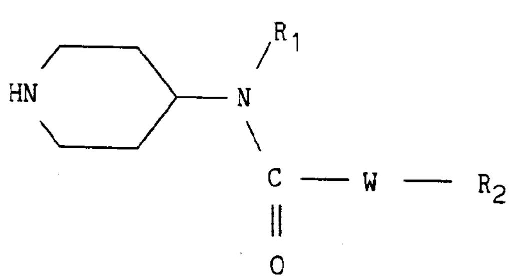

($III_A$)

dans laquelle $R_1$ a la même signification que pour la formule I et W et $R_2$ ont les mêmes significations que pour la formule IX, peuvent être préparés à partir de la 1-benzyl 4-oxo pipéridine. Ce composé est soumis à

l'action d'une amine de formule XIV :

$H_2N-R_1$ (XIV)

dans laquelle $R_1$ a la même signification que pour la formule I,
puis, à l'action du borohydrure de sodium, en solution dans l'isopropanol pour former les composés de formule XV :

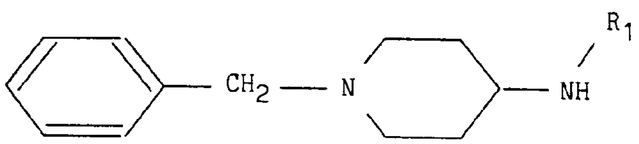

(XV)

dans laquelle la signification de $R_1$ reste identique à celle donnée pour la formule I.

Les composés de formule XV sont ensuite soumis à l'action d'un composé de formule IX, en présence de triéthanolamine dans le chlorure de méthylène pour former les composés de formule XVI :

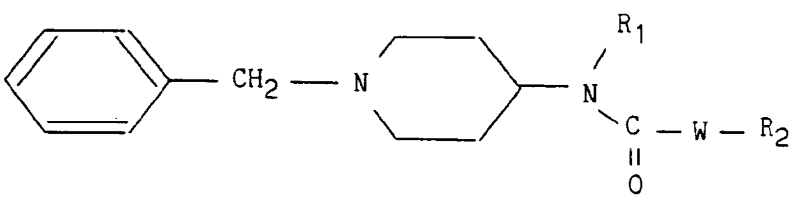

(XVI)

dans laquelle $R_1$ a la même signification que pour la formule I et $R_2$ et W ont les mêmes significations que pour la formule IX.

Ces derniers composés sont ensuite soumis à une hydrogénation catalytique pour donner les composés attendus.

Les dérivés de la pipéridine de formule $V_A$ :

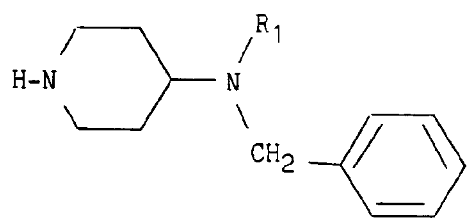

($V_A$)

dans laquelle $R_1$ a la même signification que pour la formule I, sont obtenus à partir de la 1-acétyl 4-oxo pipéridine. On fait réagir ce composé avec une amine de formule XVII :

$H_2N-R_1$ (XVII)

dans laquelle la signification de $R_1$ est identique à celle donnée pour la formule I et ensuite on soumet le milieu réactionnel à une hydrogénation catalytique pour obtenir les composés de formule XVIII :

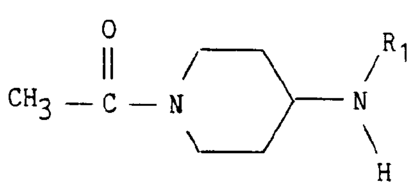

(XVIII)

dans laquelle la signification de $R_1$ reste identique à celle donnée ci-dessus.

Les composés de formule XVIII sont ensuite condensés avec le chlorure de benzyle dans un alcool, en présence de carbonate de sodium, pour donner les composés de formule XIX :

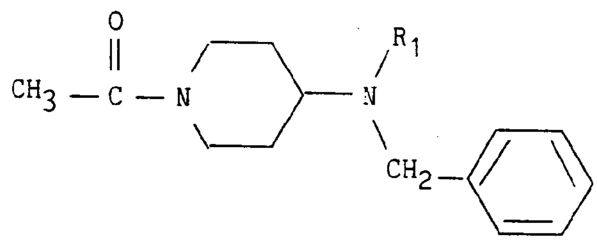

(XIX)

dans laquelle $R_1$ a la même signification que pour la formule I. Ces derniers composés sont soumis ensuite à l'action de l'acide chlorhydrique, dans le méthanol pour donner les composés de formule $V_A$.

Les isomères optiques des composés de la formule I, qui font aussi l'objet de la présente invention, peuvent être obtenus par des méthodes classiques (salification avec un acide optiquement actif).

Parmi les acides pharmaceutiquement acceptables pour la préparation des sels d'addition des composés de formule générale I, on peut citer les acides phosphorique, chlorhydrique, citrique, iodhydrique, oxalique, maléïque, sulfurique, tartrique, mandélique, fumarique, méthanesulfonique, etc...

Les composés de l'invention, ainsi que leurs sels d'addition sont doués de propriétés pharmacologiques fort intéréssantes. Les essais pharmacologiques ont démontré que les composés de l'invention se comportent comme de très puissants antagonistes des récepteurs de sérotonine 5-$HT_{1A}$ avec une activité antagoniste au niveau du système nerveux central.

De plus, certains d'entre eux sont de bons ligands du récepteur sigma.

Les composés de l'invention trouvent donc leur application dans le traitement du stress (Neuropharmac.,(1989), Vol.25, N°5, p.471-476), de la migraine (T.I.P.S., (1989), Vol.10, pp. 200-204), de l'anxiété, de la dépression, de la schizophrénie et de la douleur [Pharmacology and Toxicology, (1989), 64, p.3-5 ; Drugs of the future, (1988), 13. N°5, p. 429 -437 ; J.Neurol. Transm., (1988), 74, p. 195 - 198].

Les composés actifs au niveau des récepteurs 5-$HT_{1A}$ peuvent aussi modifier le comportement alimentaire et sexuel (J. of Receptor Research, (1988),8 ,p.59-81).

Les propriétés pharmacologiques des ligands sigma sont illustrées notamment, de façon générale, par : WALKER J. MICHAEL et Coll., Pharmacological Reviews (1990), 42 (4), 355-402, et dans le domaine de l'analgésie par : Trends in Neurosci (1987), 10, 444-446 ; Clin. Neuropharmacol. (1988), 11, 105 et Mol. Pharmacol. (1987), 32, 772-784.

Les ligands sigma servent aussi à moduler l'action d'une variété de neurotransmetteurs - cf Eur. J Pharmacol. (1988), 149, 399-400.

La 1,3-ditolylguanidine et ses dérivés ont été utilisés dans le diagnostic et le traitement des hallucinations associées aux psychoses mentales cf Trends in Neurosci. (1988), 11, 37-40.

L'invention s'étend aussi aux compositions pharmaceutiques renfermant comme principe actif au moins un composé de formule générale I, ou l'un de ses sels avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, suppositoires, solutions injectables ou buvables.

La posologie peut varier largement en fonction de l'âge, du poids du patient, de la nature et de la sévérité de l'affection, ainsi que de la voie d'administration. D'une manière générale, la posologie unitaire s'échelonnera entre 0,1 et 100 mg, et la posologie journalière utilisable en thérapeutique humaine entre 0,1 et 500 mg. La voie d'administraton préférée est la voie orale ou parentérale.

Les exemples suivants, donnés à titre non limitatif, illustrent l'invention.

Les points de fusion ont été mesurés, sauf mention contraire, selon la technique Micro-Kofler.

Les spectres de résonance magnétique nucléaire du proton des composés de formule générale I, ont été enregistrés selon le cas à 200 ou à 400 MHz et sont indiqués dans le Tableau I.

**EXEMPLE 1**

**Chlorhydrate de [1-(benzocyclobutén-1-yl méthyl) pipérid-4-yl] N-méthyl carbamate de méthyle**

Stade A

N-(1-benzyl pipérid-4-yl) N-méthyl carbamate de méthyle

A 33 g du dichlorhydrate de 1-benzyl 4-méthylaminopipéridine dans 300 ml de chlorure de méthylène et 50,5 ml de triéthylamine refroidis à 5 °C, on coule goutte à goutte 11 g de chloroformiate de méthyle. On laisse 1 heure à 0 °C, puis transvase en ampoule et lave à l'eau. Après séchage et évaporation, on obtient le produit attendu.
- Rendement : 71 %

Spectre de résonance magnétique nucléaire du proton (solvant $CDCl_3$) :
7,3 ppm, 5H, **m** ; 3,7 à 4,1 ppm, 1H, **m** ; 3,7 ppm, 3H, **s** ; 3,5 ppm, 2H, s_; 2,95 ppm, 2H, **d** ; 2,75 ppm, 3H, **s** ; 2,1 ppm, 2H, **t** ; 1,5 à 1,9 ppm, 4H, **m**.

Stade B

N-pipérid-4-yl N-méthyl carbamate de méthyle

Le carbamate décrit au stade précédent est soumis à l'hydogénation sous 5 Kg à 50°C dans l'éthanol en présence de Pd/C à 5 % et une quantité adéquate d'acide chlorhydrique concentré pour obtenir le chlorhydrate correspondant. Après passage à la base en présence d'éther éthylique et de soude à 40 %, on obtient le composé attendu.
- Rendement : 53 %
- Spectre de résonance magnétique nucléaire du proton (solvant $CDCl_3$) :
  4,1 ppm, 1H, **m** ; 3,7 ppm, 3H, **s** ; 3,15 ppm, 2H, **m** ; 2,8 ppm, 3H, **d** ; 2,6 à 2,9 ppm, 2H, **m** ; 1,5 à 1,7 ppm, 4H, **m** ; 1,75 ppm, **1H échangeable**

Stade C

7,3 g de 1-iodométhyl benzocyclobutène (préparé selon le procédé décrit dans la demande de brevet FR 89.14571 du 07 novembre 1989) et 5,1 g du composé obtenu au stade B dans 100 ml de diméthylformamide sont portés à 60°C sous agitation pendant 6 heures.

Evaporer le solvant, reprendre à l'eau, extraire à l'éther éthylique et épuiser la phase éthérée à l'acide chlorhydrique N. Basifier à froid et extraire à l'éther éthylique pour obtenir le N-[1-(benzocyclobutén-1-yl méthyl) pipérid-4-yl] N-méthyl carbamate de méthyle.
- Rendement : 43 %

3,7 g de cette base dissous dans 30 ml d'éthanol sont salifiés par 10 ml d'éther chlorhydrique 3 N pour obtenir le chlorhydrate correspondant.
- Rendement : 59 %
- Point de fusion : > 260°C
- Analyse élémentaire

| | Théorie | Trouvé |
|---|---|---|
| C % | 62,86 | 62,85 62,51 |
| H % | 7,76 | 7,95 7,70 |
| N % | 8,62 | 8,57 8,43 |
| Cl % | 10,91 | 10,97 10,54 |

**EXEMPLE 2**

**Chlorhydrate de N-[1-(benzocyclobutén-1-yl méthyl) pipérid-4-yl] N-méthyl carbamate de propyle**

Ce composé a été obtenu selon le procédé décrit dans l'exemple 1 mais en utilisant au stade A, le chloroformiate de propyle au lieu du chloroformiate de méthyle.

- Rendement (base) : 28 %
- Rendement (sel) : 73 %
- Point de fusion : 248 - 250°C
- Analyse élémentaire :

| | Théorie | Trouvé | |
|---|---|---|---|
| C % | 64,67 | 64,35 | 64,18 |
| H % | 8,28 | 8,48 | 8,40 |
| N % | 7,94 | 7,95 | 7,93 |
| Cl % | 10,05 | 10,17 | 10,05 |

**EXEMPLE 3**

**Chlorhydrate de N-[1-(benzocyclobutén-1-yl méthyl) pipérid-4-yl] N-méthyl pentafluoropropionamide**

Ce composé a été préparé selon le procédé décrit dans l'exemple 1 mais en utilisant au stade A, le chlorure de pentafluoropropionyle au lieu du chloroformiate de méthyle.

- Rendement (sel) : 10,5 %
- Point de fusion : 246 - 248°C
- Analyse élémentaire :

| | Théorie | Trouvé | |
|---|---|---|---|
| C % | 52,37 | 52,56 | 52,56 |
| H % | 5,37 | 5,54 | 5,53 |
| N % | 6,79 | 6,78 | 6,68 |
| Cl % | 8,59 | 8,85 | 8,49 |

**EXEMPLE 4**

**Chlorhydrate de N-[1-(benzocyclobutén-1-yl méthyl) pipérid-4-yl] N-méthyl carbamate de tertiobutyle**

Stade A

N-(1-benzyl pipérid-4-yl) N-méthyl carbamate de tertiobutyle.

A 27,8 g de N-(1-benzylpipérid-4-yl)N-méthylbutanamide dans 200 ml de dioxane et 200 ml de soude 1 N, couler 24 g de pyrocarbonate de tertiobutyle en maintenant la température à 5°C. Laisser 1 heure à 5°C, puis extraire à l'éther éthylique.

L'huile résiduelle est soumise à une chromatographie flash en utilisant comme solvant, un mélange de chlorure de méthylène et d'acétate d'éthyle (80:20 V/V) pour obtenir le composé attendu.

- Rendement : 66 %
- Spectre de résonance magnétique nucléaire du proton (solvant $CDCl_3$) :
  7,3 ppm, 5H, **m** ; 4,2 à 3,7 ppm, 1H, **s** ; 3,5 ppm, 2H, **s** ; 2,95 ppm, 2H, **m** ; 2,75 ppm, 3H, **s** ; 2,05 ppm, 2H, **m** ; 1,9 à 1,55 ppm, 4H, **m** ; 1,45 ppm, 9H, **s**.

Stade B

N-pipérid-4-yl N-méthyl carbamate de tertiobutyle

20 g du composé obtenu au stade précédent dans 200 ml d'éthanol et 3,6 g d'acide acétique avec 2 g de Pd/C à 5 % sont soumis à une hydrogénation sous 5 Kg à 50°C.

Après évaporation et déplacement de l'acétate par la lessive de soude en présence d'éther éthylique, décanter, sécher et évaporer pour obtenir le composé attendu.

- Rendement : 71 %
- Point de fusion : < 50°C
- Spectre de résonance magnétique nucléaire du proton (solvant $CDCl_3$) :
  4 ppm, 1H, **m** ; 3,1 ppm, 2H, **m** ; 2,7 ppm, 3H, **s** ; 2,6 ppm, 2H, **m** ; 1,4 à 1,7 ppm, 4H, **m** ; 1,45 ppm, 9H, **s** ; 1,65 ppm, **1H échangeable**.

Stade C

Le chlorhydrate de N-[1-(benzocyclobutén-1-yl méthyl) pipérid-4-yl] N-méthyl carbamate de tertiobutyle est obtenu selon le procédé décrit dans l'exemple 1 stade C à partir du 1-iodométhyl benzocyclobutène et du N-pipérid-4-yl N-méthyl carbamate de tertiobutyle.

- Rendement : 60 %
- Point de fusion : > 260°C
- Analyse élémentaire :

| | Théorie | Trouvé |
|---|---|---|
| C % | 65,47 | 64,97 |
| H % | 8,52 | 8,63 |
| N % | 7,63 | 7,53 |
| Cl % | 9,66 | 9,84 |

**EXEMPLE 5**

**Chlorhydrate de N-[1-benzocyclobutén-1-yl méthyl) pipérid-4-yl] N-méthyl carbamate de phényle**

Ce composé a été préparé selon le procédé décrit dans l'exemple 1 mais en utilisant au stade A le chloroformiate de phényle au lieu du chlorure de propionyle.

- Rendement : 21 %
- Point de fusion : 262 - 264°C
- Analyse élémentaire :

| | Théorie | Trouvé | |
|---|---|---|---|
| C % | 68,29 | 67,71 | 67,76 |
| H % | 7,03 | 7,52 | 7,08 |
| N % | 7,24 | 7,01 | 6,98 |
| Cl % | 9,16 | 9,16 | 9,09 |

**EXEMPLE 6**

**Chlorhydrate de N-[1-(benzocyclobutén-1-yl méthyl) pipérid-4-yl] N-éthyl carbamate d'éthyle**

Ce composé a été préparé selon le procédé décrit dans l'exemple 1 mais à partir du dichlorhydrate de 1-benzyl 4-éthylamino pipéridine et en remplaçant au stade A le chloroformiate de méthyle par le chloroformiate d'éthyle.

- Rendement : 13 %
- Point de fusion : 258 - 260°C
- Analyse élémentaire :

| | Théorie | Trouvé | |
|---|---|---|---|
| C % | 64,67 | 64,42 | 64,51 |
| H % | 8,28 | 8,69 | 8,69 |
| N % | 7,94 | 7,83 | 7,75 |
| Cl % | 10,05 | 10,25 | 9,96 |

**EXEMPLE 7**

**Chlorhydrate de N-[1-(benzocyclobutén-1-yl méthyl) pipérid-4-yl] N-éthyl carbamate de méthyle.**

Ce composé a été préparé selon le procédé décrit dans l'exemple 1 en utilisant au stade A la 1-benzyl 4-éthylamino pipéridine et le chloroformiate de méthyle.

- Rendement : 13 %
- Point de fusion : 258 - 260°C
- Analyse élémentaire :

| | Théorie | Trouvé | |
|---|---|---|---|
| C % | 63,80 | 63,92 | 63,71 |
| H % | 8,03 | 8,34 | 8,18 |
| N % | 8,27 | 8,07 | 8,10 |
| Cl % | 10,46 | 10,57 | 10,39 |

**EXEMPLE 8**

**Fumarate de N-[1-(benzocyclobutén-1-yl méthyl) pipérid-4-yl] N-méthyl carbamate d'isobutyle**

La base a été préparée aussi selon le procédé décrit dans l'exemple 1 en utilisant au stade A le chloroformiate d'isobutyle au lieu du chloroformiate de méthyle.

Le N-[1-(benzocyclobutén-1-yl méthyl) pipérid-4-yl] N-méthyl carbamate d'isobutyle ainsi obtenu, est ensuite salifié par une quantité adéquate d'acide fumarique dans l'éthanol.

- Rendement : 27 %
- Point de fusion : 190 - 192°C
- Analyse élémentaire :

| | Théorie | Trouvé | |
|---|---|---|---|
| C % | 64,55 | 64,60 | 64,50 |
| H % | 7,67 | 7,94 | 7,82 |
| N % | 6,29 | 6,31 | 6,35 |

**EXEMPLE 9**

**Chlorhydrate de N-[1-(benzocyclobutén-1-yl méthyl) pipérid-4-yl] N-méthyl carbamate de 2-méthoxyéthyle**

Ce composé a été préparé selon le procédé décrit dans l'exemple 4 (stades B et C) mais en utilisant au stade B le N-(1-benzyl pipérid-4-yl) N-méthyl carbamate de 2-méthoxyéthyle. Ce dernier composé est obtenu selon le procédé décrit dans l'exemple 1 stade B en utilisant le chloroformiate de méthoxyéthyle au lieu du chlorure de propionyle.

- Rendement : 14 %
- Point de fusion : 210 - 212°C
- Analyse élémentaire :

| | Théorie | Trouvé | |
|---|---|---|---|
| C % | 61,86 | 61,81 | 61,69 |
| H % | 7,92 | 7,92 | 8,03 |
| N % | 7,59 | 7,51 | 7,23 |
| Cl % | 9,61 | 9,78 | 9,53 |

**EXEMPLE 10**

**Chlorhydrate de N-[benzocyclobutén-1-yl méthyl) pipérid-4-yl] N-méthyl carbamate de cyclohexyle**

Ce composé a été préparé selon le procédé décrit dans l'exemple 4 stades B et C mais en utilisant au stade B le N-(1-benzyl pipérid-4-yl) N-méthyl carbamate de cyclohexyle. Ce dernier composé est obtenu selon le procédé décrit dans l'exemple 1 stade B en utilisant le chloroformiate d'hexyle.

- Rendement : 13 %
- Point de fusion : 262 - 264°C
- Analyse élémentaire :

| | Théorie | Trouvé | |
|---|---|---|---|
| C % | 67,24 | 66,77 | 66,72 |
| H % | 8,46 | 8,53 | 8,64 |
| N % | 7,13 | 7,35 | 7,33 |
| Cl % | 9,02 | 8,48 | 8,57 |

## EXEMPLE 11

**Chlorhydrate de N-[1-(benzocyclobutén-1-yl méthyl) pipérid-4-yl] N-méthyl carbamate de benzyle**

Stade A

1-acétyl 4-méthylamino pipéridine

A 14,4 g de 1-acétyl 4-oxo pipéridine dans 100 ml d'éthanol, sont ajoutés 9,9 g de méthylamine dans 50 ml d'éthanol. Hydrogéner ensuite en présence d'oxyde de platine, à température ambiante et à pression atmosphérique. Filtrer. Evaporer.

- Rendement : 98 %
- Spectre de résonance magnétique nucléaire du proton (solvant $CDCl_3$) :
  4,4 à 3,7 ppm, 2H , **m** ; 3,1 à 2,7 ppm, 2H, **s** ; 2,6 ppm, 1H, **m** ; 2,45 ppm, 3H, **s** ; 2,05 ppm, 3H, **s** ; 1,35 et 1,2 ppm, 4H, **m** ; 1,7 ppm, **1H échangeable**

Stade B

N-(1-acétyl pipérid-4-yl) N-méthyl carbamate de benzyle

L'amine obtenue au stade A est traitée avec le chloroformiate de benzyle pour obtenir le carbamate attendu.

- Rendement : 54 %
- Spectre de résonance magnétique nucléaire du proton (solvant $CDCl_3$):
  7,35 ppm, 5H, **m** ; 5,1 ppm, 2H, **s** ; 4,75 ppm, 1H, **m** ; 4,2 ppm, 1H, **m** ; 3,85 ppm, 1H, **m** ; 3,1 ppm, 1H, **t** ; 2,8 ppm, 3H, **s** ; 2,55 ppm, 1H, **t** ; 2,1 ppm, 3H, **s** ; 1,8 à 1,4 ppm, 4H, **m**.

Stade C

Chlorhydrate de N-pipérid-4-yl N-méthyl carbamate de benzyle

14,5 g du composé obtenu au stade B sont portés à reflux avec 50 ml de méthanol et 30 ml d'acide chlorhydrique 6N pendant 20 heures. Après évaporation on obtient le chlorhydrate du produit attendu.

- Rendement : 83 %
- Point de fusion : 203 - 205°C
- Spectre de résonance magnétique nucléaire du proton (solvant $CDCl_3$) : 7,4 ppm, 5H, **m** ; 5,15 ppm, 2H, **s** ; 4,3 ppm, 2H, **m** ; 3,6 ppm, 2H, **d** ; 2,95 ppm, 2H, **m** ; 2,85 ppm, 3H, **s** ; 2,25 ppm , 2H, **m** ; 1,85 ppm, 2H, **d**.

Stade D

Le chlorhydrate de N-[1-(benzocyclobutén-1-yl méthyl) pipérid-4-yl] N-éthyl carbamate de benzyle a été préparé à partir du chlorhydrate de N-pipérid-4-yl N-méthyl carbamate de benzyle et du 1-iodométhyl benzocyclobutène, selon le procédé décrit dans l'exemple 1 stade C, en présence de triéthylamine.

- Rendement : 22,5 %
- Point de fusion : 210 - 212°C

- Analyse élémentaire :

| | Théorie | Trouvé |
|---|---|---|
| C % | 68,90 | 68,84 68,97 |
| H % | 7,29 | 7,43 7,55 |
| N % | 6,99 | 7,06 7,08 |
| Cl % | 8,84 | 9,00 8,79 |

**EXEMPLE 12**

**Chlorhydrate de N-{1-[(1-méthyl benzocyclobutén-1-yl) méthyl] pipérid-4-yl} N-méthyl propionamide**

Ce composé a été obtenu à partir du 1-méthyl 1-iodométhyl benzocyclobutène (préparé selon le procédé décrit dans la demande de brevet FR 89.14571 du 07 novembre 1989), et le N-pipérid-4-yl N-méthyl propionamide, selon le procédé décrit dans l'exemple 1 stade C.

- Rendement : 14,5 %
- Point de fusion : 222°C
- Analyse élémentaire :

| | Théorie | Trouvé |
|---|---|---|
| C % | 67,74 | 67,43 |
| H % | 8,68 | 8,59 |
| N % | 8,31 | 8,24 |
| Cl % | 10,52 | 10,65 |

**EXEMPLE 13**

**Chlorhydrate de N-[1-(benzocyclobutén-1-yl méthyl) pipérid-4-yl] N-éthyl acétamide**

3,3 g de N-pipérid-4-yl N-éthyl acétamide et 2.8 g de tosylate de 1-méthyl benzocyclobutène (préparé selon le procédé décrit dans J.A.C.S., (1975), 154, p. 347) sont portés à reflux sous azote dans 16 ml de toluène pendant 20 heures. Après refroidissement, le milieu réactionnel est évaporé à siccité et repris à l'éther éthylique. Après extraction à l'acide chlorhydrique N, basifier en présence d'éther éthylique et laver la phase organique à l'eau. Sécher sur sulfate de magnésium, et évaporer sous vide, pour obtenir le N-[1-(benzocyclobutén-1-yl méthyl) pipérid-4-yl] N-éthyl acétamide. Le chlorhydrate correspondant est obtenu dans l'acétate d'éthyle en présence d'éther chlorhydrique.

- Rendement : 20,7 %
- Point de fusion : 265 - 267°C
- Analyse élémentaire :

| | Théorie | Trouvé |
|---|---|---|
| C % | 66,96 | 67,08 |
| H % | 8,43 | 8,55 |
| N % | 8,68 | 8,59 |
| Cl % | 10,98 | 11,02 |

**EXEMPLE 14**

**Chlorhydrate de N-[1-(benzocyclobutén-1-yl méthyl) pipérid-4-yl] N-méthyl propionamide**

Ce composé a été préparé selon le procédé décrit dans l'exemple 13 en utilisant le N-pipérid-4-yl N-méthyl propionamide comme amide.

- Rendement : 28 %
- Point de fusion : 302 - 306°C
- Analyse élémentaire :

| | Théorie | Trouvé |
|---|---|---|
| C % | 66,96 | 66,67 67,00 |
| H % | 8,43 | 8,24 8,10 |
| N % | 8,68 | 8,62 8,66 |
| Cl % | 10,98 | 10,87 10,87 |

**EXEMPLE 15**

**Chlorhydrate de N-[1-(benzocyclobutén-1-yl méthyl) pipérid-4-yl N-méthyl carbamate d'éthyle**

Ce composé a été préparé selon le procédé décrit dans l'exemple 13 en utilisant le N-pipérid-4-yl N-méthyl carbamate d'éthyle au lieu du N-pipérid-4-yl N-éthyl acétamide.

- Rendement : 58 %
- Point de fusion : 305 -313°C
- Analyse élémentaire :

| | Théorie | Trouvé |
|---|---|---|
| C % | 63,80 | 63,55 63,84 |
| H % | 8,03 | 7,80 8,00 |
| N % | 8,27 | 8,44 8,49 |
| Cl % | 10,46 | 10,78 10,78 |

**EXEMPLE 16**

**Chlorhydrate de N-[1-(benzocyclobutén-1-yl méthyl) pipérid-4-yl] N-méthyl butyramide**

4 g de N-pipérid-4-yl N-méthylbutanamide et 3,1 g de tosylate de 1-méthyl benzocyclobutène sont portés à reflux sous azote dans 20 ml de toluène pendant 18 heures. Après refroidissement, le milieu réactionnel est évaporé à siccité et repris à l'éther éthylique.

Après extraction à l'acide chlorhydrique N, basifier en présence d'éther éthylique, laver la phase organique à l'eau et sécher sur sulfate de magnésium. Après évaporation sous vide du solvant, la base obtenue est reprise par de l'acétate d'éthyle et traitée par de l'éther chlorhydrique pour obtenir le composé attendu.

- Rendement : 47 %
- Point de fusion : 238°C
- Analyse élémentaire :

| | Théorie | Trouvé | |
|---|---|---|---|
| C % | 67,74 | 67,48 | 67,55 |
| H % | 8,68 | 8,83 | 8,86 |
| N % | 8,31 | 8,24 | 8,28 |
| Cl % | 10,52 | 10,59 | 10,45 |

**EXEMPLE 17**

**Chlorhydrate de N-[1-(benzocyclobutén-1-yl méthyl) pipérid-4-yl] N-méthyl carbamate d'allyle**

Stade A

N-(1-acétyl pipérid-4-yl) N-benzyl méthylamine

Porter à reflux pendant une nuit un mélange contenant 0,5 mole du composé obtenu dans l'exemple 11 stade A, 0,5 M de chlorure de benzyle et 1 M de carbonate de sodium dans 650 ml d'éthanol. Filtrer le précipité formé, évaporer le solvant, reprendre par l'acide chlorhydrique 1N, extraire par l'éther éthylique, basifier par la lessive de soude et extraire à l'acétate d'éthyle pour obtenir le composé attendu.

- Rendement : 65 %
- Spectre de résonance magnétique nucléaire du proton (solvant $CDCl_3$) :
  7,25 ppm, 5H, m ; 4,6 ppm, 1H, m ; 3,8 ppm, 1H, m ; 3,55 ppm, 2H, s ; 3 ppm , 1H, td ; 2,7 à 2,4 ppm, 2H, m ; 2,2 ppm, 3H, s ; 2,05 ppm, 3H, s ; 1,85 ppm, 2H, m ; 1,55 ppm, 2H, m.

Stade B

4-(N-benzyl N-méthyl amino) pipéridine

80 g du composé obtenu au stade précédent en solution dans 375 ml de méthanol sont traités par 97,5 ml d'acide chlorhydrique concentré et 97,5 ml d'eau pendant 2 heures à reflux. Evaporer et basifier la solution réactionnelle et extraire à l'éther éthylique pour obtenir le composé attendu.
Rendement : 85,5 %

Stade C

1-(benzocyclobutén-1-yl carbonyl) 4-(N-benzyl N-méthyl amino) pipéridine

Ajouter à une solution de 36,2 g d'acide benzocyclobutén-1-yl carboxylique dans 400 ml de chlorure de méthylène, 41 g de carbonyl diimidazole. Laisser 4 heures sous agitation, et ajouter le composé obtenu au

stade précédent en solution dans le chlorure de méthylène. Porter à reflux pendant 72 heures. Diluer le milieu réactionnel avec 1500 ml d'éther éthylique et extraire 3 fois par 100 ml d'acide chlorhydrique 0,1 N. Eliminer les phases aqueuses et réextraire la phase organique trois fois, avec 100 ml d'acide chlorhydrique 1N. Les phases aqueuses acides sont ensuite basifiées et extraites à l'éther éthylique pour obtenir le composé attendu.

- Rendement : 34 %
- Spectre de résonance magnétique nucléaire du proton (solvant $CDCl_3$) :
  7,4 à 7 ppm, 9H, **m** ; 4,65 ppm, 1H, **m** ; 4,45 ppm, 1H, **m** ; 4,15 ppm, à 1H, **m** ; 3,6 ppm, 2H, **s** ; 3,6 à 3,3 ppm, 2H, **m** ; 3,15 ppm, 1H, **m** ; 2,8 à 2,5 ppm, 1H+1H, **m** ; 2,25 ppm, 3H, **s** ; 2 ppm, 2H, **m** ; 1,8 à 1,4 ppm, 2H, **m**.

Stade D

1-{[4-(N-benzyl N-méthylamino) pipérid-1-yl] méthyl} benzocyclobutène

Couler 28 g du composé obtenu au stade C en solution dans 150 ml de tétrahydrofurane sur une suspension de 3,2 g d'hydrure de lithium et d'aluminium dans 50 ml de tétrahydrofurane. Porter à reflux pendant 3 heures, traiter selon l'usage, filtrer et évaporer la phase organique pour isoler le composé attendu.

- Rendement : 84,5 %
- Spectre de résonance magnétique nucléaire du proton (solvant $CDCl_3$) :
  7,3 à 6,9 ppm, 9H, **m** ; 3,7 ppm, 1H, **m** ; 3,55 ppm, 2H, **s** ; 3,35 ppm, 1H, **dd** 3,1 ppm, 2H, **m** ; 2,8 ppm, 1H+1H, **m** ; 2,6 à 2,4 ppm, 2H, **m** ; 2,2 ppm, 3H, **s** 2,05 ppm, 2H, **m** ; 1,9 à 1,6 ppm, 4H, **m**.

Stade E

1-[(4-méthylamino pipérid-1-yl) méthyl] benzocyclobutène

22 g du composé obtenu au stade D dans 220 ml d'éthanol et 4,2 ml d'acide acétique contenant 2,2 g de Pd/C à 5 % sont hydrogénés sous 5 Atm. à 50°C. Eliminer le catalyseur par filtration, évaporer, concrétiser par agitation avec 20 ml d'éther éthylique, puis alcaliniser par l'hydroxyde de sodium à 20 % pour relarguer le produit attendu, en présence de 200 ml d'éther éthylique.

- Rendement : 77 %
- Spectre de résonance magnétique nucléaire du proton (solvant $CDCl_3$) :
  7,4 à 7 ppm, 4H, **m** ; 3,75 à 3,6 ppm, 1H, **m** ; 3,45 à 3,25 ppm, 1H, **m** ; 3,05 à 2,9 ppm, 2H **m** ; 3 à 2,5 ppm, 1H+1H **m** ; 2,45 ppm, 3H, **s** ; 2 à 2 ppm, 2H, **m** ; 2,0 à 1,8 ppm, 2H, **m** ; 1,55 à 13 ppm, 2H, **m** ; 2 à 1,7 ppm, **1H échangeable** ; 2,8 ppm, 1H, **m** ; 2,5 à 2,3 ppm, 1H, **m**.

Stade F

Couler sur 1,8 ml de triéthylamine et 3 g du composé obtenu au stade précédent en solution dans 30 ml de benzène, 1,4 ml de chloroformiate d'allyle en solution dans 5 ml de benzène. Diluer à l'éther éthylique, laver à l'eau, sécher, évaporer.
Diluer le résidu obtenu dans 10 ml d'acétate -d'éthyle et ajouter 3,3 ml d'éther chlorhydrique 3,6 N pour obtenir le chlorhydrate de N-[1-(benzocyclobutén-1-yl méthyl) pipérid-4-yl] N-méthyl carbamate d'allyle.
Rendement : 53 %
Point de fusion : 233 - 235°C
Analyse élémentaire :

| | Théorie | Trouvé | |
|---|---|---|---|
| C % | 65,04 | 64,84 | 64,90 |
| H % | 7,76 | 7,66 | 7,87 |
| N % | 7,98 | 7,98 | 7,86 |
| Cl % | 10,10 | 10,05 | 9,88 |

**EXEMPLE 18**

**Chlorhydrate de N-[1-(benzocyclobutén-1-yl méthyl) pipérid-4-yl] N-méthyl acrylamide**

Ce composé a été obtenu selon le procédé décrit dans l'exemple 17 stade F mais en remplaçant le chloroformiate d'allyle par le chlorure d'acryloyle.

- Rendement : 24 %
- Point de fusion : 230 - 232°C
- Analyse élémentaire :

| | Théorie | Trouvé |
|---|---|---|
| C % | 67,38 | 67,04 |
| H % | 7,85 | 7,80 |
| N % | 8,73 | 8,66 |
| Cl % | 11,05 | 11,14 |

**EXEMPLE 19**

**Fumarate de N-{1-[(3,4-méthylènedioxy benzocyclobutén-1-yl) méthyl] pipérid-4-yl) N-méthyl carbamate d'éthyle**

Stade A

Acide 2-cyano 3-(2,3-méthylènedioxy phényl) propén-2-oïque

Mélanger 160 g de 2,3-méthylènedioxybenzaldéhyde, 90,52 g d'acide cyanoacétique, 149,2 ml de pyridine et 13,6 g d'acétate d'ammonium. Porter à reflux pendant 12 heures dans 944 ml de toluène.

A l'aide d'un dean-starck, récupérer 17 ml d'eau puis laisser une nuit à température ambiante. Filtrer le précipité, le reprendre dans 600 ml d'acide chlorhydrique à 18 %. Filtrer et laver à l'eau, jusqu'à neutralité.

Extraire la phase organique avec une solution saturée de bicarbonate de sodium. Garder le précipité formé puis, acidifier la phase aqueuse et extraire au chlorure de méthylène. Extraire la phase organique avec une solution saturée de bicarbonate de sodium. Garder le précipité formé, qui est l'acide attendu.

- Rendement : 47,5 %
- Point de fusion : 230°C
- Spectre de résonance magnétique nucléaire du proton (solvant DMSO-$d_6$) :
  8,2 ppm, 1H s ; 7,7 ppm, 1H, d ; 7,15 ppm, 1H, d ; 7,0 ppm, 1H, t ; 6,2 ppm 2H, s ; 3,5 ppm **1H échangeable**.

Stade B

Acide 2-cyano 3-(2,3 méthylènedioxy phényl) propanoïque

60,2 g de borohydrure de sodium sont ajoutés à un mélange contenant 110 g de l'acide obtenu au stade A et 404,5 ml d'une solution saturée de bicarbonate de sodium à 18°C. Laisser pendant 48 heures. Reprendre par l'eau, laver à l'éther éthylique puis acidifier par l'acide chlorhydrique jusqu'à pH 2. Extraire au chlorure de méthylène, puis laver la phase organique à l'eau jusqu'à neutralité, sécher pour obtenir le composé attendu.

- Rendement : 55 %
- Point de fusion : 118°C
- Spectre de résonance magnétique nucléaire du proton (solvant $CDCl_3$ + DMSO-$d_6$) :
  8,8 ppm **1H échangeable** ; 6,8 ppm, 3H, **m** ; 6,0 ppm, 2H, **m** ; 3,85 ppm, 1H, **dd** ; 3,3 ppm, 1H, **dd** ; 3,10 ppm, 1H, **dd**.

Stade C

2-cyano 1-(2,3-méthylènedioxyphényl) éthane.

Mélanger 60,6 g d'acide préparé au stade B et 115 ml de NN-diméthylacétamide puis amener à 150°C pendant 2 heures environ. Laisser refroidir. Reprendre par l'eau, extraire à l'éther éthylique. Laver les phases éthérées par une solution de bicarbonate de sodium puis par l'eau. Sécher pour obtenir le composé attendu sous forme d'huile.

- Rendement : 87 %
- Spectre de résonance magnétique nucléaire du proton (solvant $CDCl_3$) :
  6,9 à 6,65 ppm, 3H, **m** ; 5,95 ppm, 2H, **s** ; 2,95 ppm, 2H, **t** ; 2,65 ppm 2H **t**.

Stade D

1-(6-bromo 2,3-méthylèndioxyohényl) 2-cyano éthane

A 53,6 g de nitrile obtenu au stade C, dissous dans 179 ml d'acide acétique, couler à 18°C 16,4 ml de brome en solution dans 35 ml d'acide acétique. Agiter pendant 1 heure, puis laisser la nuit à température ambiante. Hydrolyser sur 31,5 g d'acétate de potassium en solution dans 150 ml d'eau et 196,5 g de glace. Extraire à l'éther éthylique, laver la phase éthérée plusieurs fois à l'eau. Sécher.

- Rendement : 24 %
- Point de fusion : 60 - 65°C
- Spectre de résonance magnétique nucléaire du proton (solvant $CDCl_3$)
  7,0 ppm, 1H, **d** ; 6,65 ppm, 1H, **d** ; 6,0 ppm, 2H, **s** ; 3,05 ppm, 2H, **t** ; 2,65 ppm,2H, **t**.

Stade E

1-cyano 3,4-méthylènedioxybenzocyclobutène

Ajouter 15 g du composé obtenu au stade précédent sur 0,1 mole d'amidure de sodium dans l'ammoniac liquide. Laisser 15 minutes puis neutraliser avec 10,2 g de chlorure d'ammonium. Laisser s'évaporer l'ammoniac puis reprendre par l'eau et l'éther éthylique. Filtrer les insolubles. Décanter le filtrat. Réextraire la phase aqueuse par de l'éther éthylique. Rassembler les phases organiques, les laver par l'acide chlorhydrique N puis sécher sur sulfate de manganèse.

- Rendement : 57 %
- Point de fusion : 80°C
- Spectre de résonance magnétique nucléaire du proton (solvant $CDCl_3$) :
  6,6 à 6,9 ppm, 2H, **d ;** 5,95 ppm, 2H, **s** ; 4,2 ppm, 1H, **m** ; 3,55 ppm, 2H, **m**

Stade F

Acide 3,4-méthylènedioxy benzocyclobutén-1-yl carboxylique

5,8 g du composé précédemment préparé sont agités à température ambiante, pendant une nuit, dans une solution éthanolique de potasse (6,7 g dans 48 ml d'éthanol). Ajouter ensuite 9 ml d'eau et amener à reflux pendant 4 heures. Concentrer, reprendre par l'eau, laver plusieurs fois à l'éther éthylique et acidifier jusqu'à pH 1 avec l'acide chlorhydrique concentré puis extraire à l'éther éthylique. Sécher.

- Rendement : 99 %
- Point de fusion : 125°C
- Spectre de résonance magnétique nucléaire du proton (solvant $CDCl_3$) :
  6,7 ppm, 2H, **2d** ; 5,95 ppm, 2H, **s** ; 4,3 ppm, 1H, **t** ; 3,45 ppm, 2H, **d**

Stade G

1-hydroxyméthyl 3,4-méthylènedioxybenzocyclobutène

Sous atmosphère d'azote, ajouter 7,4 g d'hydrure de lithium et d'aluminum sur 100 ml d'éther éthylique, puis couler, goutte à goutte, 15 g du composé préparé au stade F, dissous dans 300 ml d'éther éthylique. Après

la fin de l'addition, amener à reflux pendant 3 heures. Hydrolyser l'excès d'hydrure, filtrer, laver plusieurs fois à l'éther éthylique. Concentrer. On obtient l'alcool sous forme d'huile.

- Rendement : 87 %
- Spectre de résonance magnétique nucléaire du proton (solvant $CDCl_3$) :
  6,65 ppm, 2H, **2d** ; 5,9 ppm, 2H, **s** ; 3,9 ppm, 2H, **m** ; 3,65 ppm, 1H, **m** 3,25 ppm, 1H, **dd** ; 2,9 ppm, 1H, **dd** ; 1,5 ppm, **1H échangeable**.

Stade H

Tosylate de 1-méthyl 3,4-méthylènedioxy benzocyclobutène

Ajouter à 0°C, 19 g de chlorure de para-toluène sulfonyle sur 12,1 g d'alcool obtenu au stade précédent, dissous dans 84 ml de pyridine. Laisser agiter pendant 48 heures à température ambiante. Concentrer. Reprendre par l'eau, filtrer, laver plusieurs fois à l'eau puis à l'acide chlorhydrique 1 N. Sécher.

- Rendement : 82 %
- Point de fusion : 102°C
- Spectre de résonance magnétique nucléaire du proton (solvant DMSO-$d_6$) :
  7,75 ppm, 2H, **d** ; 7,45 ppm, 2H, **d** ; 6,75 ppm, 1H, **d** ; 6,55 ppm, 1H, **d** ; 5,95 ppm, 2H, **s** ; 4,25 ppm, 2H, **m** ; 3,7 ppm, 1H, **m** ; 3,3 à 3,1 ppm, 1H, **2d** 2,7 à 2,8 ppm, 1H, **2d** ; 2,4 ppm, 3H, **s**.

Stade I

4 g du composé obtenu au stade H, 2,24 g de N-pipérid-4-y N-méthyl Carbamate d'éthyle et 1,7 ml de triéthanolamine sont amenés à reflux dans 40 ml de toluène pendant 24 heures. Concentrer le milieu réactionnel, reprendre par l'acétate d'éthyle, laver à l'eau puis extraire à l'acide chlorhydrique 1 N. Basifier avec de la soude concentrée puis extraire au chlorure de méthylène. Sécher pour obtenir le N-{1-[(3,4-méthylènedioxy benzocyclobutén-1-yl) méthyl] pipérid-4-yl} N-méthyl carbamate d'éthyle. Rajouter 23,5 ml d'une solution éthanolique à 2 % en acide fumarique pour obtenir le sel attendu.

- Rendement : 17 %
- Point de fusion : 138 - 142°C
- Analyse élémentaire :

| | Théorie | Trouvé |
|---|---|---|
| C % | 59,73 | 59,45 |
| H % | 6,54 | 6,41 |
| N % | 6,06 | 5,82 |

**EXEMPLE 20**

**Chlorhydrate de N-{[1-(3,4-méthylènedioxy benzocyclobutén-1-yl) méthyl] pipérid-4-yl} N-méthyl propionamide**

Ce composé a été préparé selon le procédé décrit dans l'exemple 19 stade I mais en utilisant le N-pipérid-4-yl N-méthyl propionamide au lieu de N-pipérid-4-yl N-méthyl carbamoylate de méthyle. Pour la salification il a été utilisé l'éther chlorhydrique.

- Rendement : 36 %
- Point de fusion : 208 - 211°C
- Analyse élémentaire :

| | Théorie | Trouvé | |
|---|---|---|---|
| C % | 62,20 | 62,10 | 61,83 |
| H % | 7,42 | 7,58 | 7,46 |
| N % | 7,64 | 7,51 | 7,39 |
| Cl % | 9,66 | 9,39 | 9,36 |

**EXEMPLE 21**

**N-(1-indan-2-yl pipérid-4-yl) N-méthyl propionamide**

Stade A

p-Toluène sulfonate d'indanyle

A 30 g de 2-indanol dissous dans 75 ml de pyridine, refroidis à 0°C, ajouter 47,4 g de chlorure de tosyle. Laisser 3 heures à cette température puis une nuit à température ambiante. Couler ensuite la solution réactionnelle sur 450 ml d'acide chlorhydrique 2,6 N, filtrer et laver à l'eau pour obtenir le composé attendu.

- Point de fusion : 117°C
- Spectre de résonance magnétique nucléaire du proton (solvant $CDCl_3$) : 7,8 ppm, 2H, d ; 7,35 ppm, 2H, d ; 7,15 ppm, 4H, s ; 5,3 ppm, 1H, m ; 2,95 à 3,35 ppm, 4H, m ; 2,5 ppm, 3H, s.

Stade B

Mélanger 4 g du composé obtenu au stade précédent, 2,95 g du composé décrit dans l'exemple 1 stade C, ainsi que 3 ml de NN-diisopropyléthylamine dans 40 ml de toluène et amener à reflux pendant 24 heures.

Concentrer et reprendre le milieu réactionnel par de l'éther éthylique, laver à l'eau et extraire par l'acide chlorhydrique.

Basifier la phase aqueuse et extraire à l'éther éthylique. Sécher, concentrer. Recristalliser dans 7 ml d'acétate d'éthyle.

- Rendement : 30 %
- Point de fusion : 126 - 129°C
- Analyse élémentaire :

| | Théorie | Trouvé | |
|---|---|---|---|
| C % | 75,48 | 75,76 | 75,19 |
| H % | 9,15 | 8,96 | 9,19 |
| N % | 9,78 | 9,80 | 9,70 |

**EXEMPLE 22**

**Chlorhydrate de N-{1-(benzocyclobutén-1-yl éthyl) pipérid-4-yl} N - méthyl propionamide**

Ce composé a été préparé à partir de 1-bromoéthyl benzocyclobutène et de N-pipérid-4-yl N-méthyl propionamide selon le procédé décrit dans l'exemple 20.

- Rendement : 55 %
- Point de fusion : 224 - 230°C

- Analyse élémentaire :

| | Théorie | Trouvé |
|---|---|---|
| C % | 67,74 | 67,59 |
| H % | 8,68 | 8,66 |
| N % | 8,31 | 8,13 |
| Cl % | 10,52 | 10,45 |

**EXEMPLE 23**

**Chlorhydrate de 1-[(1-benzocyclobutén-1-yl méthyl) pipérid-4-yl] 1-méthyl 3-phényl urée**

Ajouter 0,02 mole d'isocyanate de phényle en solution dans 20 ml d'éther éthylique sur une solution de 1-benzocyclobutén-1-yl 4-méthylamino pipéridine en solution dans l'éther éthylique, en maintenant la température entre 0°C et 5°C. Laisser ensuite 1 heure à cette température puis filtrer le précipité obtenu de façon à avoir l'urée attendue. Dissoudre ensuite ce composé dans l'acétonitrile et salifier avec une quantité adéquate d'éther chlorhydrique.

- Rendement : 80 %
- Point de fusion : > 260°C
- Analyse élémentaire :

| | Théorie | Trouvé |
|---|---|---|
| C % | 68,47 | 68,38 68,08 |
| H % | 7,31 | 7,31 7,26 |
| N % | 10,89 | 11,06 11,10 |
| Cl % | 9,19 | 8,79 8,58 |

**EXEMPLE 24**

**Chlorhydrate de 1-[(1-benzocyclobutén-1-yl méthyl) pipérid-4-yl] 3-éthyl 1-méthyl urée**

Ce composé a été préparé selon le procédé décrit dans l'exemple 23 mais en remplaçant l'isocyanate de phényle par l'isocyanate d'éthyle.

- Rendement : 65 %
- Point de fusion : > 260°C
- Analyse élémentaire :

| | Théorie | Trouvé |
|---|---|---|
| C % | 63,98 | 63,99  63,63 |
| H % | 8,35 | 8,40  8,36 |
| N % | 12,44 | 12,57  12,67 |
| Cl % | 10,49 | 10,47  10,23 |

**EXEMPLE 25**

**1-[(1-benzocyclobutén-1-yl méthyl) pipérid-4-yl] 3-benzyl 1-méthyl urée**

Ce composé a été préparé selon le procédé décrit dans l'exemple 23 mais en remplaçant l'isocyanate de phényle par l'isocyanate de benzyle.

- Rendement : 85 %
- Point de fusion : 148 - 150°C
- Analyse élémentaire :

| | Théorie | Trouvé |
|---|---|---|
| C % | 76,00 | 75,56  75,92 |
| H % | 8,04 | 8,01  7,99 |
| N % | 11,56 | 11,55  11,69 |

**EXEMPLE 26**

Chlorhydrate de N-[1-(benzocyclobutén-1-yl méthyl) pipérid-4-yl] N-méthyl methacrylamide.

A 4,6 g de 1-[(4-methylamino pipérid-1-yl) méthyl] benzocyclobutène (préparé au stade E de l'exemple 17) et 2,02 g de triéthylamine dans 50 ml de benzène, on coule, lentement, à température ambiante, 2,08 g de chlorure de méthacryloyle.

Le mélange réactionnel est laissé une nuit à température ambiante puis transvasé dans une ampoule à brome et épuisé avec une solution normale d'HCl. Les phases aqueuses réunies sont basifiées à froid puis extraites à l'éther.

Après chromatographie flash ($CH_3COOC_2H_5$), on obtient 1,6 g de N-[1-(benzocyclobutén-1-yl méthyl) pipérid-4-yl] N-méthyl méthacrylamide, sous forme d'huile.

Rendement : 26 %

Spectre de résonnance magnétique nucléaire du proton (solvant $CDCl_3$) :

7,3 à 7 ppm, 4H, **m** ;5, 15 à 5 ppm, 2H, **2m** ; 4,5 et 3,7 ppm, 1 H, **2m** ; 3,7 ppm, 1H, **m** ; 3,4 ppm, 1H, **dd** ; 3,1 ppm, 2H, **d** ; 2,9 à 2,7 ppm, 2H, **dd+m** ; 2,6 ppm, 1H, **dd** ; 2,2 ppm, 2H, **m** ; 2 ppm, 3H, **s** ; 2 à 1,5 ppm, 4H, **m**.

Le chlorhydrate de N-[1-(benzocyclobutén-1-yl méthyl) pipérid-4-yl] N-méthyl méthacrylamide a été obtenu par addition de la quantité stoechiométrique d'éther chlorhydrique 3,6 N à 1,6 g de base précédemment préparée dissoute dans 10 ml d'acétonitrile. Après filtration et recristallisation du méthanol on obtient 0,6 g du composé attendu.

Rendement : 34 %

Point de fusion : > 260°C (K)

Analyse élémentaire :

| | Théorie | Trouvé |
|---|---|---|
| C % | 68,14 | 67,90  67,91 |
| H % | 8,13 | 8,06  8,12 |
| N % | 8,36 | 7,92  8,03 |
| Cl % | 10,59 | 10,87  10,53 |

**EXEMPLE 27**

**N-[1-(benzocyclobutén-1-yl méthyl) pipérid-4-yl] N-méthyl formamide.**

A une solution de 32 ml d'acide formique à 88 % et de 3 g de 1-[(4-méthylamino pipérid-1-yl) méthyl] benzocyclobutène (cf stade E de l'exemple 17), préchauffée à 40°C, on ajoute goutte à goutte 11 ml d'anhydride acétique.

Le mélange réactionnel est maintenu une nuit sous agitation, puis on l'évapore, reprend à l'eau glacée, basifie à froid et extrait à l'éther. Après évaporation et recristallisation dans 15 ml d'éther isopropylique, on obtient 5,3 g de N-[1-(benzocyclobutén-l-yl méthyl) pipérid-4-yl] N-méthyl formamide.
Rendement : 48 %
Point de fusion : 86-88°C
Analyse élémentaire :

| | Théorie | Trouvé |
|---|---|---|
| C % | 74,38 | 74,21  74,34 |
| H % | 8,58 | 8,57  8,63 |
| N % | 10,84 | 10,79  10,68 |

**EXEMPLE 28**

**Chlorhydrate de N-[1-(benzocyclobutén-1-yl méthyl) pipérid -4-yl] N-méthyl carbamoylate d'oxa-2 propyle (R,S)**

Stade A

En opérant à partir de 1-benzyl 4-méthylamino pipéridine et de

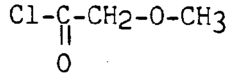

on obtient le N-(1-benzyl pipérid-4-yl) N-méthyl carbamoylate d'oxa-2 propyle.
Rendement : 87 %
Spectre de résonance magnétique nucléaire du proton (solvant $CDCl_3$) :
7,3 ppm, 5H, m ; 4,5 et 3,5 ppm, 1H, 2m ; 4,10 et 4 ppm, 2H, 2s ; 3,5 ppm, 2H, m ; 3,4 ppm, 3H, s ; 3 ppm, 2H, m ; 2,8 ppm, 3H, s ; 2,35 à 1,5 ppm, 6H, m.

Stade B

22 g de la base précédemment obtenue, dans 220 ml d'éthanol et 4,8 ml d'acide acétique sont hydrogénés

sous une pression de 5 kg d'hydrogène à 50°C en présence de 2,2 g d'hydroxyde de palladium. On filtre ensuite le catalyseur, évapore la solution, reprend le résidu par 500 ml de chlorure de méhtylène et basifie à froid avec 60 ml de soude à 20 %. Après décantation, séchage et évaporation, on obtient 9,3 g de N-(pipérid-4-yl) N-méthyl carbamoylate d'oxa-2 propyle, sous forme d'huile.
Rendement : 62 %
Spectre de résonance magnétique nucléaire du proton (solvant $CDCl_3$) :
4,55 à 3,6 ppm, 1H, **2m** ; 4,1 ppm, 2H, **2s** ; 2,45 ppm, 3H, **2s** ; 3,2 ppm, 2H, **m** ; 2,85 ppm, 3H, **2s** ; 2,75 ppm, 2H, **m** ; 1,9 à 1,55 ppm, 4H, **m** ; 3,8 ppm, 1H échangé $D_2O$, **s**.

Stade C

On chauffe à reflux sous agitation pendant une nuit 1,8 g de N-(pipérid-4-yl) N-méthyl carbamoylate d'oxa-2 propyle précédemment obtenu, 1,1 g de triéthylamine et 2,8 g de tosylate de 1-hydroxyméthyl benzocyclobutane dans 50 ml de toluène.

On évapore, reprend à l'éther et extrait avec une solution normale d'HCl.

Les phases aqueuses sont basifiées à froid et extraites à l'acétate d'éthyle. On obtient ainsi 1,6 g de N-[1-(benzocyclobutén-1-yl méthyl) pipérid-4-yl] N-méthyl carbamoylate d'oxa-2 propyle.
Rendement : 14 %
- Spectre de résonance magnétique nucléaire du proton (solvant $CDCl_3$) : 7,25 à 7 ppm, 4H, **m** ; 4,6 à 4,4 et 3,7 à 3,5 ppm, 1H, **m** ; 4,1 ppm, 2H, **s** ; 3,7 ppm, 1H, **m** ; 3,5 à 3,2 ppm, 4H, **s+m** ; 3 à 1,5 ppm, 12H, **s+5m** ; 3,1 ppm, 2H, m.

Stade D

Le chlorhydrate de N-[1-(benzocyclobutén-1-yl méthyl) pipérid-4-yl] N-méthyl carbamoylate d'oxa-2 propyle est obtenu par addition de la quantité stoechimétrique d'HCl 3 N dans l'éther à 1,4 g de la base préparée au stade C, dans 5 ml d'acétonitrile.

Après filtration et séchage, on obtient 1 g du chlorhydrate attendu.
Rendement : 77 %
Point de fusion : 204-206°C (K)
Analyse élémentaire :

| | Théorie | Trouvé |
|---|---|---|
| C % | 63,80 | 63,52 |
| H % | 8,03 | 7,87 |
| N % | 8,27 | 8,18 |
| Cl % | 10,46 | 10,38 |

corrigé de 0,7 % d'$H_2O$

**EXEMPLE 29**

**Chlorhydrate de N-[1-(benzocyclobutén-1-yl méthyl) pipérid-4-yl] N-méthyl carbamate de vinyle (R,S)**

En opérant comme décrit dans l'exemple 26 à partir de 3 g de 1-[(4-méthylamino pipérid-1-yl) méthyl] benzocyclobutène (préparé au stade E de l'exemple 17) et du chloroformiate de vinyle, on obtient le N-[1-(benzocyclobutén-1-yl méthyl) pipérid-4-yl] N-méthyl carbamate de vinyle (R,S) qui mis en solution dans 5 ml d'acétonitrile est salifié par une solution 3,6 N d'HCl dans l'éther, pour donner 1,3 g de chlorhydrate de N-[1-(benzocyclobutén-1-yl méthyl) pipérid-4-yl] N-méthyl carbamate de vinyle (R,S).
Rendement : 30 %
Point de fusion : 232-234 °C
Analyse élémentaire :

| | Théorie | Trouvé | |
|---|---|---|---|
| C % | 64,18 | 63,89 | 63,80 |
| H % | 7,48 | 7,34 | 7,77 |
| N % | 8,32 | 8,29 | 8,17 |
| Cl % | 10,52 | 10,82 | 10,64 |

**EXEMPLE 30**

**Fumarate de N-[1-(indan-2-yl) pipérid-4-yl] N-méthyl carbamate d'isobutyle** :

Un mélange de 3,71 g de N-(pipérid-4-yl) N-méthyl carbamate d'isobutyle (préparé dans l'exemple 8), 4,9 ml de triéthylamine, 50 ml de toluène et 5 g de p.toluène sulfonate d'indan-2-yle (préparé selon l'exemple 21 stade A) est maintenu à reflux pendant 24 heures.

Le précipité est filtré et lavé plusieurs fois au toluène. Le filtrat est ensuite lavé plusieurs fois à l'eau, séché et concentré. L'huile obtenue est reprise dans une solution d'acide fumarique à 2 % dans l'éthanol (mole à mole). Après concentration et recristallisation dans 10 ml d'éthanol on obtient le fumarate de N-[1-(indan-2-yl) pipérid-4-yl] N-méthyl carbamate d'isobutyle.

Rendement : 14 %

Point de fusion : 230-234 °C avec sublimation vers 180-185°C

Analyse élémentaire :

| | Théorie | Trouvé | |
|---|---|---|---|
| C % | 64,55 | 64,79 | 64,67 |
| H % | 7,67 | 7,71 | 7,62 |
| N % | 6,27 | 6,11 | 6,18 |

**EXEMPLE 31**

N-[1-(indan-2-yl) pipérid-4-yl] N-méthyl carbamate de propyle :

Un mélange de 3,47 g de N-(pipérid-4-yl) N-méthyl carbamate de propyle (préparé dans l'exemple 2) ; 4,9 ml de triéthylamine, 50 ml de toluène et 5 g de p.toluènesulfonate d'indan-2-yle (préparé selon l'exemple 31 stade A) est maintenu à reflux pendant 24 heures.

On ajoute ensuite 100 ml d'eau à ce mélange réactionnel, décante et extrait la phase organique par une solution normale d'HCl. La phase aqueuse acide est ensuite basifiée par une solution normale de soude, puis extraite au $CH_2Cl_2$ et séchée sur $MgSO_4$. Le solide obtenu est recristalisé dans 10 ml d'éther isopropylique.

Rendement : 42 %

Point de fusion : 73-76 °C

Analyse élémentaire :

| | Théorie | Trouvé |
|---|---|---|
| C % | 72,12 | 71,76 |
| H % | 8,92 | 8,79 |
| N % | 8,85 | 9,04 |

TABLEAU I

COMPOSES DE FORMULE GENERALE I

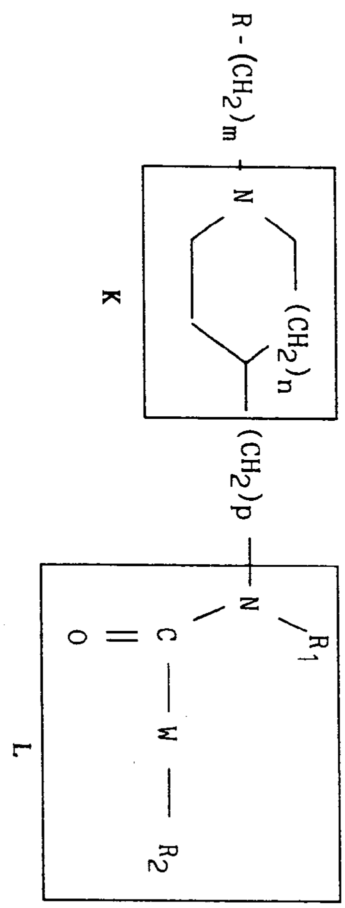

(I)

T A B L E A U I suite 2

| EXEMPLE | R | m | K | p | L | Spectre RMN (solvant) |
|---|---|---|---|---|---|---|
| 7 | 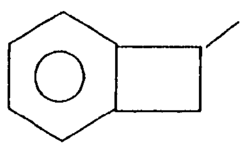 | 1 | 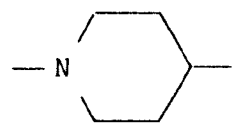 | 0 | 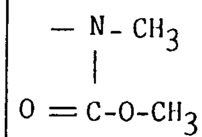 | RMN-$^1H$ ($CDCl_3$) base<br>7,3 à 7 ppm, 4H, **m** ; 4,4 ppm, 1H, **m** ; 4,2 ppm, 1H, **m** ; 3,8 à 3,4 ppm, 6H, **m** ; 3,3 à 3,0 ppm, 2H, **m** ; 3 à 2,6 ppm, 8H, **m** ;1,85 ppm, 2H, **m** ; 13 à 12,7 ppm, **1H échangeable** |

T A B L E A U I suite 3

| EXEMPLE | R | m | K | p | L | Spectre RMN (solvant) |
|---|---|---|---|---|---|---|
| 2 | 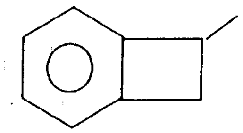 | 1 | 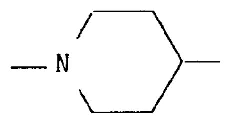 | 0 | 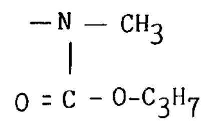 | RMN-$^1$H ($CDCl_3$) sel<br>7,4 à 7 ppm, 4H, **m** ; 4,4 ppm, 1H, **m** ; 4,3 à 3,9 ppm, 3H, **m+t** ; 3,9 à 3,3 ppm, 3H, **m+m** ; 3,3 à 3 ppm, 2H, **m+m** ; 2,85 ppm 3H, **s** ; 3 à 2,5 ppm, 3H, **m+m** ; 2 à 1,5 ppm, 6H, **m+m+m** ; 0,95 ppm, 3H, **t** ; 12,75 ppm, **1H échangeable** |
| 3 | 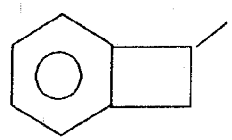 | 1 | 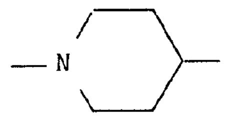 | 0 | 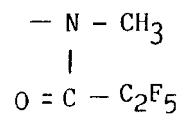 | RMN-$^1$H (DMSO-$d_6$) sel<br>7,3 à 7 ppm, 4H, **m** ; 4,5 et 4,2 ppm,1H , **2m** ; 3,9 ppm, 1H, **m** ; 3,5 ppm, 1H, **m** ; 3,5 à 3 ppm, 7H, 4**m** ; 2,8 et 3 ppm 3H, **2s** 2,1 à 2,6 ppm, 2H, **m** ; 1,85 ppm, 2H, **m** ; 10,75 ppm, **1H échangeable** |
| 4 | 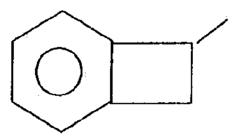 | 1 | 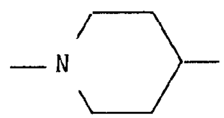 | 0 | 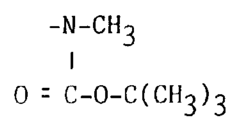 | RMN-$^1$H ($CDCl_3$ + DMSO)<br>7,3 à 7 ppm, 4H, **m** ; 4,50 à 4,05 ppm 2H, **m** ; 2,8 ppm, 3H, **s** ; 1,85 ppm, 2H, **d** 1,45 ppm, 9H, **s** ; 3,8 à 2,4 ppm, 10H, **m** ; 13 à 12 ppm, **1H échangeable** |
| 5 | 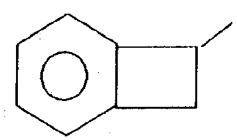 | 1 | 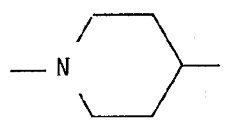 | 0 | 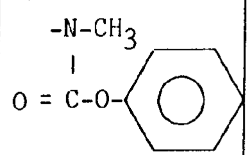 | RMN-$^1$H (DMSO-$d_6$)<br>7,5 à 7 ppm, 9H, **m** ; 4,3 ppm, 1H, **m** ; 4 ppm,1H, **m** ; 3,7 à 3 ppm, 8H, **4m** ; 2,9 ppm, 3H, **m** ; 2,5 à 2,2 ppm, 2H, **m** ; 1,9 ppm, 2H, **m** ; 10,9 ppm, **1H échangeable** |

TABLEAU I suite 4

| EXEMPLE | R | m | K | p | L | Spectre RMN (solvant) |
|---|---|---|---|---|---|---|
| 6 | 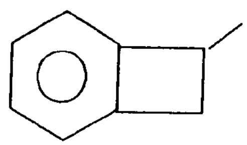 | 1 | 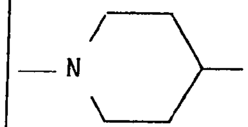 | 0 | — N – $C_2H_5$<br>\|<br>O = C – O-$C_2H_5$ | RMN-$^1$H (DMSO-$d_6$)<br>7,2 ppm, 4H, **m** ; 4,2 à 3,3 ppm, 4H, **m** ; 3,7 à 3 ppm, 10H, **m** ; 1,8 ppm, 4H, **m** ; 1,2 ppm, 3H, **t** ; 1,05 ppm, 3H, **t** ; 10,9 ppm, **1H échangeable** |
| 7 | 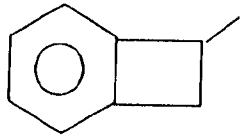 | 1 | 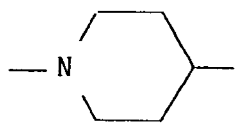 | 0 | — N – $C_2H_5$<br>\|<br>O = C – O-$CH_3$ | RMN-$^1$H (DMSO-$d_6$) sel<br>7,3 à 7 ppm, 4H, **m** ; 4,1 à 3,8 ppm, 1H+1H **m+m** ; 3,6 ppm, 3H, **s** ; 3,6 à 3 ppm, 1H+1H +2H+4H+2H, **m+m+m+m+m** ; 2,3 ppm, 2H, **m** ; 1,75 ppm, 2H, **m**, 1,1 ppm, 3H, **t** ; 10,95 ppm, **1H échangeable** |
| 8 | 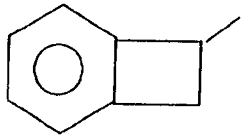 | 1 | 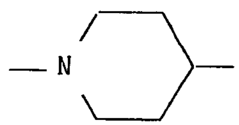 | 0 | -N- $CH_3$ $CH_3$<br>\| \|<br>O = C-O-$CH_2$-CH<br>\|<br>$CH_3$ | RMN-$^1$H (DMSO-$d_6$) sel<br>7,15 ppm, 4H, **m** ; 6,6 ppm, 2H, **s** ; 4 à 3,6 ppm, 2H, **m** ; 3,8 ppm, 2H, **d** ; 3,3 ppm, 1H, **dd** ; 3,15 ppm, 2H, **m** ; 3 à 2,6 ppm, 3H, **m** ; 2,75 ppm, 3H, **s** ; 2,3 ppm, 2H, **m** ; 2 à 1,7 ppm, 3H, **m** ; 1,6 ppm, 2H, **m** ; 0,9 ppm, 6H, **d** |

TABLEAU I suite 5

| EXEMPLE | R | m | K | p | L | Spectre RMN (solvant) |
|---|---|---|---|---|---|---|
| 9 | 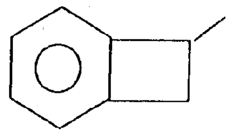 | 1 | 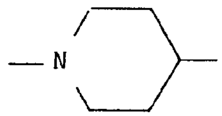 | 0 | 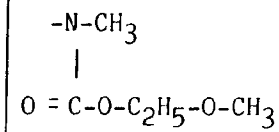 | **RMN-$^1$H ($CDCl_3$) sel** 7,3 à 7, 4H, **m** ; 4,5 à 4,1 ppm, 4H, **m+m+m** ; 3,8 à 3,3 ppm, 8H, **m+m+m+s+m** ; 3,3 à 3 ppm, 3H, **m** 3 à 2,6 ppm, 7H, **s+m+m** ; 1,8 ppm 2H, **m** ; 12,8 ppm, 1H échangeable |
| 10 | 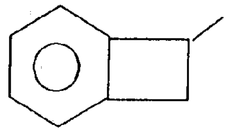 | 1 | 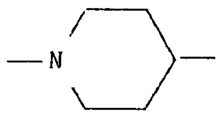 | 0 | 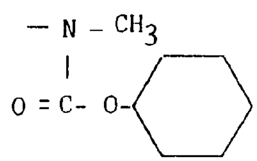 | **RMN-$^1$H (DMSO - $d_6$) sel** 7,2 ppm, 4H, **m** ; 4,55 ppm, 1H, **m** ; 4,3 à 3,8 ppm, 2H, **m+m** ; 3,7 à 2,9 ppm, 8H, **m+m+m+m** ; 2,75 ppm, 3H, **s** ; 2,2 ppm, 2H, **m** ; 1,9 à 1,2 ppm, 12H, **m** ; 10,95 ppm, 1H échangeable |
| 11 | 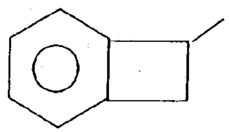 | 1 | 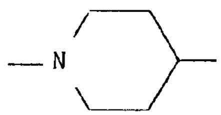 | 0 | 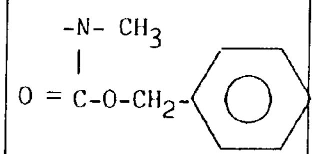 | **RMN-$^1$H (DMSO - $d_6$) sel** 7,6 à 7 ppm, 9H, **m** ; 5,1 ppm, 2H, **s** ; 4,2 ppm, 1H, **m** ; 3,95 ppm, 1H, **m** ; 3,8 à 3 ppm, 8H, **m** ; 2,8 ppm, 3H, **s** ; 2,25 ppm, 2H, **m** ; 1,8 ppm, 2H, **d** ; 11,2 ppm 1H échangeable |
| 12 | 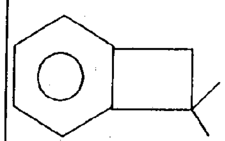 CH3 | 1 | 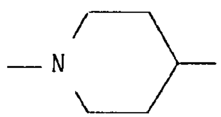 | 0 | 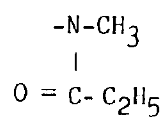 | **RMN-$^1$H ( $CDCl_3$) sel** 7,4 à 7,0 ppm, 4H+1 échangeable, **m** ; 4,8 ppm, 1H, **m** ; 3,9 à 2,6 ppm, 11H, **m** 2,3 ppm, 2H, **q** ; 1,8 ppm, 3H, **s** ; 1,9 à 1,5 ppm, 4H, **m** ; 1,15 ppm, 3H, **t** |

TABLEAU I suite 6

| EXEMPLE | R | m | K | p | L | Spectre RMN (solvant) |
|---|---|---|---|---|---|---|
| 13 | 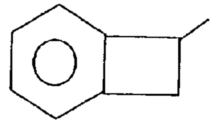 | 1 | 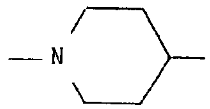 | 0 | 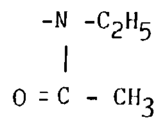 | RMN-$^{1}$H ($CDCl_3$) sel<br>7,25 ppm, 2H, m ; 7,1 ppm, 2H, m ; 4,8 ppm, 1H, m ; 4,2 ppm, 1H, m ; 3,8 - 3,1 ppm, 8H, m ; 2,9-2,7 ppm, 4H, 2m ; 2,15 ppm, 3H, s ; 1,9 ppm, 2H, m ; 1,25 ppm, 3H, t ; 12,8 ppm, 1H échangeable |
| 14 | 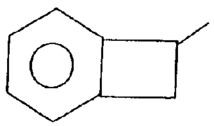 | 1 | 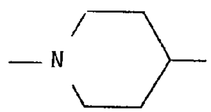 | 0 | 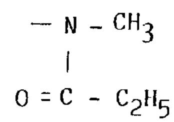 | RMN-$^{1}$H ($CDCL_3$) sel<br>7,25 ppm, 2H, m ; 7,1 ppm, 2H, m ; 4,85 ppm, 1H, m ; 4,2 ppm, 1H, m ; 3,75 ppm, 2H, m ; 3,6 ppm, 1H, dd ; 3,5 ppm, 1H, dd ; 3,25 ppm, 1H, dd ; 3,15 ppm, 1H, dd ; 3,0 à 2,6 ppm, 4H, m ; 3,0 ppm, 3H, s ; 2,35 ppm, 2H, q ; 1,8 ppm, 2H, m ; 1,15 pm, 3H, t ; 12,75 ppm, 1H échangeable |
| 15 | 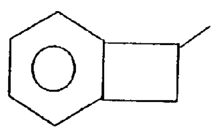 | 1 | 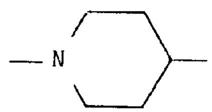 | 0 | 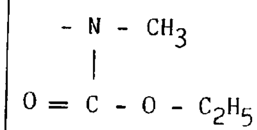 | RMN-$^{1}$H ($CDCL_3$) sel<br>7,25 ppm, 2H, m ; 7,1 ppm, 2H, m ; 4,4 ppm, 1H, m ; 4,15 ppm, 3H, m+q ; 3,75 ppm, 2H, m ; 3,6 ppm, 1H, dd ; 3,5 ppm, 1H, dd ; 3,25 ppm, 1H, d ; 3,1 ppm, 1H, dd ; 2,85 ppm, 3H, s ; 3,05-2,6 ppm, 4H, m ; 1,85 ppm, 2H, m ; 1,3 ppm, 3H, t 11,7 ppm 1H échangeable |
| 16 | 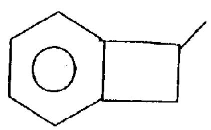 | 1 | 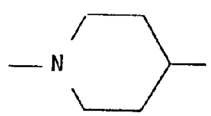 | 0 | 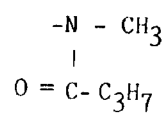 | RMN-$^{1}$H ( $CDCl_3$) sel<br>7,25 ppm, 2H, m ; 7,1 ppm, 2H, m ; 4,85 ppm, 1H, m ; 4,2 ppm, 1H, m ; 3,8-3,1 ppm, 6H, m ; 3,0 ppm, 3H, s ; 3,05-2,6 ppm, 4H, m ; 2,3 ppm, 2H, t ; 1,8 ppm, 2H, m ; 1,7 ppm, 2H, s ; 1,0 ppm, 3H, t ; 12,7 ppm, 1H échangeable |

T A B L E A U I suite 7

| EXEMPLE | R | m | K | p | L | Spectre RMN (solvant) |
|---|---|---|---|---|---|---|
| 17 | 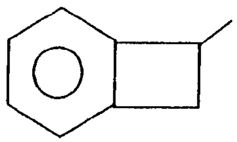 | 1 | 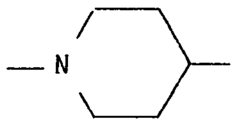 | 0 | 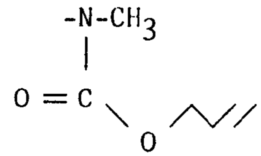 | RMN-$^1$H ($CDCl_3$) sel<br>7,3 à 7,1 ppm, 4H, m ; 6,1 à 5,8 ppm, 1H, m ; 5,4 à 5,1 ppm, 2H, m ; 4,5 ppm, 2H, d ; 4,2 ppm, 1H, m ; 3,95 ppm, 1H, m ; 3,7 à 3 ppm, 2H, m + 1H, dd + 2H, m + 2H, m + 1H, dd ; 2,75 ppm, 3H, s ; 2,4 à 2,1 ppm, 2H, m ; 1,75 ppm, 2H, m ; 10,9 ppm, 1H échangeable |
| 18 | 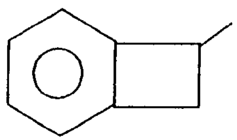 | 1 | 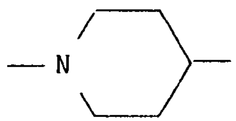 | 0 | 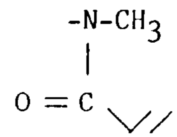 | RMN-$^1$H (DMSO-$d_6$) sel<br>7,2 ppm, 4H, m ; 6,8 ppm, 1H, m ; 6,1 à 5,7 ppm, 2H, 2d ; 4,65 à 4,2 ppm, 1H, 2m ; 3,95 ppm, 1H, m ; 3,7 à 3 ppm, 2H+1H+2H+2H, m+dd+m+m ; 2,75 à 2,5 ppm, 3H, 2s ; 2,25 ppm, 2H, m ; 1,7 ppm, 2H, m ; 10,8 ppm, 1H échangeable |
| 19 | 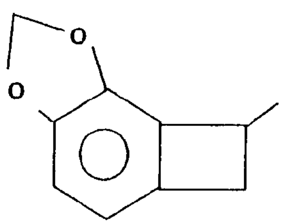 | 1 | 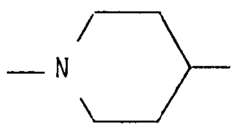 | 0 | 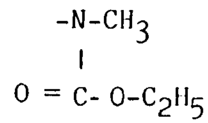 | RMN-$^1$H ( $CDCl_3$+DMSO-$d_6$) sel<br>6,8 ppm, 2H, s ; 6,7 ppm, 1H, d ; 6,55 ppm, 1H d ; 5,9 ppm, 2H, s ; 4,15 ppm, 3H, q ; 3,8 ppm, 1H, m ; 3,6-3,2 ppm, 3H, m ; 3,15-2,7, 3H, m ; 2,75 ppm, 3H, s ; 2,5 ppm, 2H, m ; 2,05 ppm, 2H, m ; 1,75 ppm, 2H, m ; 1,25 ppm, 3H, t ; 7,3 ppm, 2H échangeables |

T A B L E A U I suite 8

| EXEMPLE | R | m | K | p | L | Spectre RMN (solvant) |
|---|---|---|---|---|---|---|
| 20 | 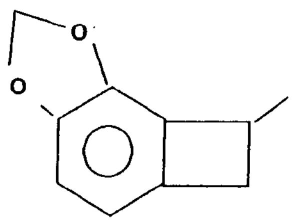 | 1 | 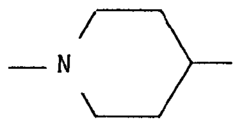 | 0 | 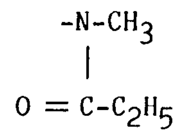 | RMN-$^1$H (DMSO-$d_6$) sel<br>6,8 ppm, 1H, d ; 6,7 ppm, 1H, d ; 6 ppm, 2H, 2s ; 4,6 à 3,5 ppm, 1H, 2m ; 3,95 ppm, 1H, m ; 3,7 ppm, 8H, m ; 2,8 ppm, 3H, 2s, 2,4 à 2 ppm, 2H+2H, q+m ; 1,9 à 1,5 ppm, 2H, m ; 1 ppm, 3H, t ; 11,5 à 11 ppm, 1H échangeable |
| 21 | 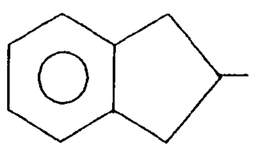 | 0 | 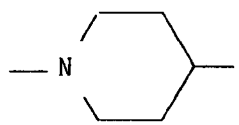 | 0 | 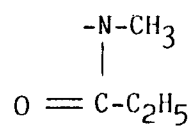 | RMN-$^1$H ($CDCl_3$) base<br>7,15 ppm, 4H, m ; 4,55 à 3,55 ppm, 1H, 2m ; 3,2 à 3 ppm, 5H, m ; 3,0 à 2,9 ppm, 2H, d ; 3,85 ppm, 3H, d ; 2,35 ppm, 2H, q ; 2,2 ppm, 2H, q ; 2,05 à 1,5 ppm, 4H, m ; 1,15 ppm, 3H, dd |

T A B L E A U I suite 9

| EXEMPLE | R | m | K | p | L | Spectre RMN (solvant) |
|---|---|---|---|---|---|---|
| 22 | 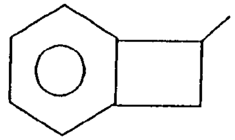 | 2 | 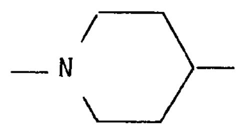 | 0 | 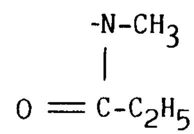 | RMN-$^{1}$H ($CDCL_3$) sel<br>7,2+7,05 ppm, 2H+2H, **m+m** ; 4,8 ppm, 1H, **m** ; 3,6 ppm, 3H, **m** ; 3,4 ppm, 1H, **dd** ; 3,1 ppm, 2H, **m** ; 2,9 ppm, 3H, **s** ; 2,45 à 2,55 ppm, 5H, **m** ; 2,35 ppm, 4H, **q+m** 1,75 ppm, 2H, **m** ; 1,1 ppm, 3H, **t** ; 12,5 ppm, **1H échangeable** |

TABLEAU I suite 10

| EXEMPLE | R | m | K | p | L | Spectre RMN (solvant) |
|---|---|---|---|---|---|---|
| 23 | 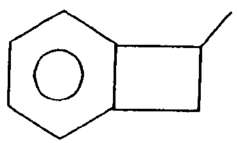 | 1 | 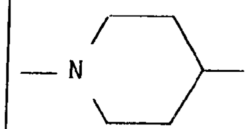 | 0 | 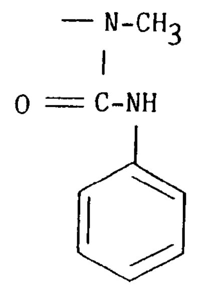 | RMN-$^{1}$H (DMSO-$d_6$) sel<br>7,5 ppm, 2H, d 7,3 à 7,1 ppm, 6H, m + 1H échangeable ; 6,95 ppm, 1H, t ; 3,95 ppm, 2H, m ; 3,7 à 2,9 ppm, 8H, m ; 2,85 ppm, 3H, s ; 2,25 à 13,75 ppm, 4H, m ; 8,4 ppm, 1H échangeable |
| 24 | 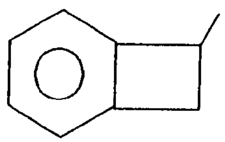 | 1 | 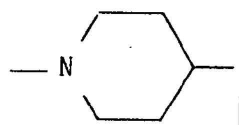 | 0 | 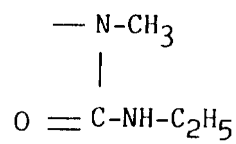 | RMN-$^{1}$H (DMSO-$d_6$) sel<br>7,2 ppm, 4H, m ; 6,35 ppm, 1H échangeable ; 4,35 ppm, 1H, m ;3,95 ppm, 1H, m ; 3,7 à 3,0 ppm, 10H, m+ dd + q + m + dd + m ; 2,7 ppm, 3H, s 2,6 à 1,65 ppm, 4H m + m ; 1,05 ppm, 3H, t ; 10,85 ppm, 1H échangeable |
| 25 | 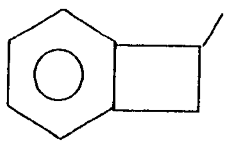 | 1 | 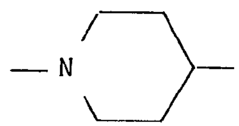 | 0 | 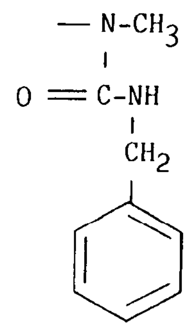 | RMN-$^{1}$H ($CDCl_3$) base<br>7,3 ppm, 5H, m ; 7,3 à 7 ppm, 4H, m ; 4,7 ppm, 1H échangeable ; 4,45 ppm, 2H, d ;4,2 ppm, 1H, m ; 3,7 ppm, 1H, m ; 3,4 ppm, 1H, dd ; 3,05 ppm, 2H, d ; 2,9 à 2,5 ppm, 3H, m + d + d ; 2,8 ppm, 3H, s 2,2 ppm 2H, t ; 1,9 à 1,6 ppm, 4H, m |

TABLEAU I suite 11

| EXEMPLE | R | m | K | p | L | Spectre RMN (solvant) |
|---|---|---|---|---|---|---|
| 26 | 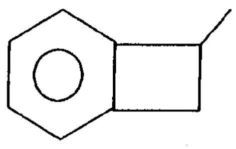 | 1 | 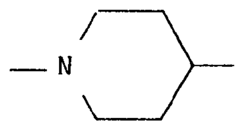 | 0 | 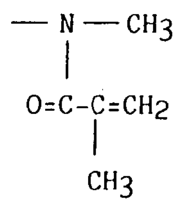 | **RMN (DMSO-$d_6$) sel**<br>7,2 ppm, 4H, **m** ; 5,1 ppm et 5 ppm, 2H, **2s** ; 3,95 ppm, 1H, **m** ; 2,55 ppm, 3H, **s** ; 3,7 à 3,1 ppm, 6H, **4m** ; 2,8 ppm, 3H, **s** ; 2,3 ppm, 2H, **m** ; 1,95 ppm, 3H, **s** ; 1,75 ppm, 2H, **m** |
| 27 | 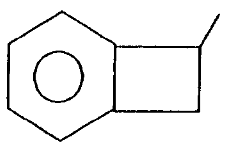 | 1 | 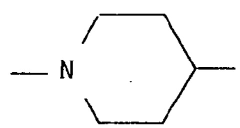 | 0 | 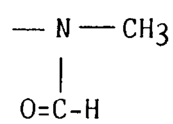 | **RMN ($CDCl_3$) base**<br>8,2 à 8,05 ppm, 1H, **2s** ; 7,2 ppm, 2H, **m** ; 7,1 ppm, 2H, **m** ; 4,3 et 3,35 ppm, 1H, **2m** ; 3,7 ppm, 1H, **m** ; 3,4 ppm, 1H, **dd** ; 3,1 ppm, 2H, **m** ; 2,9 et 2,85 ppm, 3H, **2s** ; 2,8 ppm, 2H, **m** ; 2,6 ppm, 1H, **dd** ; 2,3 à 1,1 ppm, 6H, **m** |
| 28 | 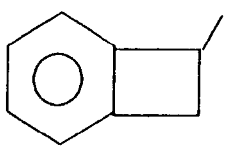 | 1 | 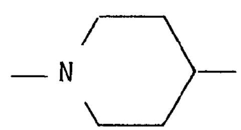 | 0 | 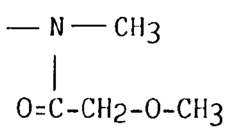 | **RMN (DMSO $d_6$) sel**<br>11,3 ppm, 1H échangé $D_2O$, **m** ; 7,3 à 7 ppm, 4H, **m** ; 4,55 et 3,9 ppm, 1H, **2m** ; 4,15 et 4,05 ppm, 2H, **2s** ; 4 ppm, 1H, **m** ; 3,7 à 3 ppm, 11H, **m+dd+m+s+dd+m** |

TABLEAU I suite 12

| EXEMPLE | R | m | K | p | L | Spectre RMN (solvant) |
|---|---|---|---|---|---|---|
| 29 | 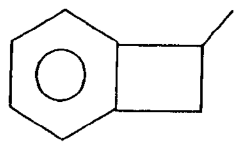 | 1 | 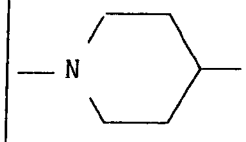 | 0 | 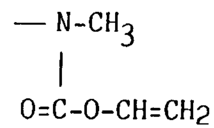 | RMN (DMSO-$d_6$) sel<br>11,05 ppm, 1H échangeable ; 7,3 à 7 ppm, 5H, 2m ; 4,8 ppm, 1H, d ; 4,05 ppm, 1H, dd ; 4,2 ppm, 1H, m ; 3,95 ppm, 1H, m ; 3,7 à 3 ppm, 8H, m+dd+2m+dd ; 2,8 ppm, 3H, s ; 2,3 ppm, 2H, m ; 1,75 ppm, 2H, m |
| 30 | 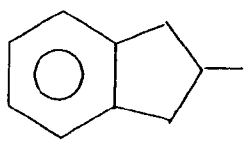 | 0 | 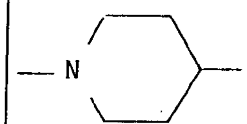 | 0 | 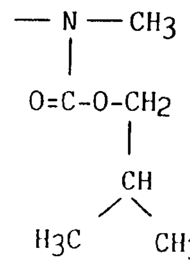 | RMN (DMSO-$d_6$) sel<br>7,15 ppm, 4H, m ; 6,6 ppm, 2H, s ; 3,85 ppm, 1H, m ; 3,75 ppm, 2H, d ; 3,25 ppm, 1H, m ; 3,15 à 2,75 ppm, 6H, m ; 2,7 ppm, 3H, s ; 2,2 ppm, 2H, m ; 1,95 à 1,5 ppm, 5H, m ; 0,9 ppm, 6H, d ; 4,5 ppm, 2H, s (très plat et très large) échangeable |
| 31 | 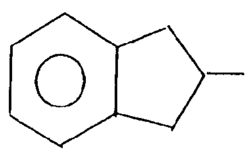 | 0 | 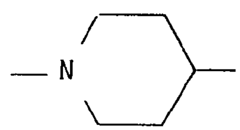 | 0 | 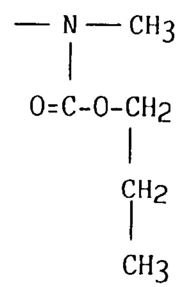 | RMN ($CDCl_3$) base<br>7,15 ppm, 4H, m ; 4,2 à 3,8 ppm, 1H, m ; 4,0 ppm, 2H, t ; 3,3 à 3,0 ppm, 5H, m ; 2,9 ppm, 2H, dd ; 2,8 ppm, 3 H, s ; 2,1 ppm, 2H, m ; 1,9 à 1,5 ppm, 6H, m ; 0,95 ppm, 3H, t |

**EXEMPLE 32 ETUDE PHARMACOLOGIQUE**

**a)** Test de "Tail-Flicks" chez le rat

L'antagonisme des récepteurs $5HT_{1A}$ des composés de l'invention a été mis en évidence selon la méthode de Millan et Coll (Neurosci Lett. (1989), 107, p.227-232).

L'injection de 8-OH-DPAT par voie sous-cutanée induit spontanément chez le rat, des mouvements de la queue ("Tail-Flicks"). Ces mouvements sont réduits de manière spécifique par les antagonistes des récepteurs $5HT_{1A}$. Les $ED_{50}$, c'est à dire les doses de composés de l'invention réduisant de 50 % l'action de 8-OH-DPAT sont donnés dans le tableau II.

TABLEAU II

| COMPOSE EXEMPLE | $ED_{50}$ mg/Kg - Voie s.c |
|---|---|
| 1 | 1,25 |
| 2 | 0,31 |
| 4 | 2,50 |
| 5 | 2,50 |
| 6 | 1,25 |
| 7 | 0,02 |
| 8 | 1,25 |
| 13 | 0,63 |
| 14 | 1,25 |
| 15 | 2,50 |
| 16 | 1,25 |
| 19 | 2,50 |
| 21 | 0,31 |
| 29 | 1,25 |
| 31 | 2,5 |

**b)** Test de binding sigma (in vitro)

L'affinité des produits de l'invention pour le récepteur sigma, dans les tissus du cerveau, est évaluée par le degré d'inhibition du binding d'un radioligand du site sigma, par l'intermédiaire d'expériences de compétition in vitro.

Le protocole expérimental utilisé est celui décrit par WEBER et al. Proc. Nat. Acad. Sci. USA, (1986), 83, 8784-8788.

Le ligand est le 1,3-di-(2-[5-$^{3}$H]tolyl) guanidine (Dupont de Nemours - 55 Ci/ m mole) à la concentration finale de 2nM.

La préparation membranaire est une suspension de microsomes de système nerveux central de cobaye utilisée à la concentration finale de 0,5 mg de prot/ml.

La fixation non spécifique est déterminée en présence de 10µm d'halopéridol.

Les résultats, présentés dans le tableau III, pour illustrer l'invention, sont exprimés en $IC_{50}$ = K.0,5 (M)

TABLEAU III

Inhibition du binding de [3H] ditolyle guanidine

| COMPOSÉ EXEMPLE | $IC_{50}$ EN M |
|---|---|
| 2 | $3.10^{-8}$ |
| 5 | $3.10^{-8}$ |
| 8 | $1.10^{-7}$ |
| 10 | $8.10^{-8}$ |
| 11 | $3.10^{-8}$ |
| 16 | $2.10^{-8}$ |
| 21 | $2.10^{-7}$ |
| 29 | $5.10^{-8}$ |
| 30 | $4.10^{-9}$ |
| 31 | $4.10^{-8}$ |

**EXEMPLE 33 COMPOSITION PHARMACEUTIQUE**

**Comprimés dosés à 5 mg de chlorhydrate de N-[1-(benzocyclobutén-1-yl méthyl) pipérid-4-yl] N-méthyl carbamate de propyle (B.M.P.M.C.P)**

| | |
|---|---|
| B.M.P.M.C.P | 5 g |
| Amidon de blé | 100 g |
| Amidon de maïs | 20 g |
| Stéarate de magnésium | 20 g |
| Talc | 20 g |

pour 1000 comprimés de 5 mg de principe actif

## Revendications

1. Composé de formule I

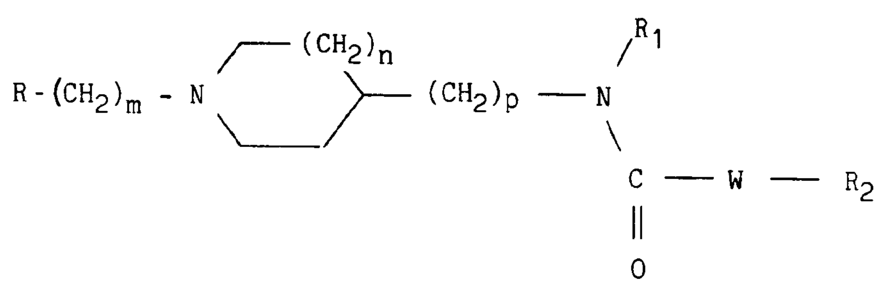

(I)

dans laquelle :

- m représente zéro, 1, 2, 3 ou 4,
- n et p représentent zéro, 1 ou 2,
- W représente un atome d'oxygène , un radical -NH-, ou une simple liaison,
- R représente :

un radical benzocyclobutén-l-yle de formule B :

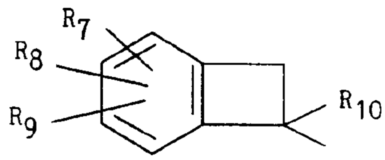

(B)

ou un radical indanyle de formule C

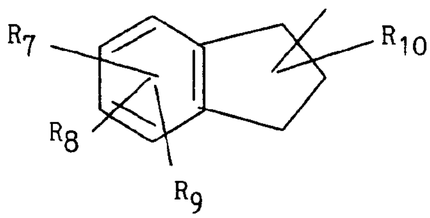

(C)

dans lesquelles :

- $R_7$, $R_8$ et $R_9$ identiques ou différents représentent chacun un atome d'hydrogène, un atome d'halogène, un radical alkyle ou alcoxy de 1 à 6 atomes de carbone, un radical alkyle polyhalogéné de 1 à 6 atomes de carbone, un radical hydroxy, ou $R_7$ et $R_8$ ou $R_8$ et $R_9$ forment ensemble un radical méthylènedioxy, un radical éthylènedioxy, un cycle furanique ou dihydrofuranique, et
- $R_{10}$ représente un atome d'hydrogène ou un radical alkyle de 1 à 6 atomes de carbone ;

- $R_1$ représente un radical alkyle de 1 à 6 atomes de carbone,
- $R_2$ représente un atome d'hydrogène, un radical alkyle de 1 à 6 atomes de carbone, un radical alcène de 2 à 6 atomes de carbone, un radical cycloalkyle de 4 à 7 atomes de carbone, un radical benzyle ou un radical phényle (chacun éventuellement substitués par un ou plusieurs atomes d'halogènes, radicaux hydroxy, ou des radicaux alkyle ou alkoxy de 1 à 6 atomes de carbone), un radical aralkyle de 7 à 12 atomes de carbone, un radical alcoxyalkyle de 2 à 7 atomes de carbone ou un radical alkyle polyhalogéné de 1 à 6 atomes de carbone,

leurs isomères optiques et leurs sels d'addition avec un acide organique ou minéral pharmaceutiquement acceptable.

2. Un composé de la revendication 1 qui est :
Le N-(1-indan-2-yl pipérid-4-yl) N-méthyl propionamide, ses isomères optiques et ses sels d'addition avec un acide minéral ou organique pharmaceutiquement acceptable.

3. Un composé de la revendication 1 qui est :
Le N-[1-(benzocyclobutén-1-yl méthyl) pipérid-4-yl] N-méthyl carbamate de propyle, ses isomères optiques et ses sels d'addition avec un acide minéral ou organique pharmaceutiquement acceptable.

4. Un composé de la revendication 1 qui est :
Le N-[1-(benzocyclobutén-1-yl méthyl) pipérid-4-yl] N-éthyl carbamate de méthyle, ses isomères optiques et ses sels d'addition avec un acide minéral ou organique pharmaceutiquement acceptable.

5. Un composé de la revendication 1 qui est :
Le N-[1-(benzocyclobutén-1-yl méthyl) pipérid-4-yl] N-éthyl acétamide, ses isomères optiques et ses sels d'addition avec un acide minéral ou organique pharmaceutiquement acceptable.

6. Un composé de la revendication 1 qui est :
Le N-[1-(benzocyclobutén-1-yl méthyl) pipérid-4-yl] N-méthyl butyramide, ses isomères optiques et ses

sels d'addition avec un acide minéral ou organique pharmaceutiquement acceptable.

**7.** Un composé de la revendication 1 qui est :
Le N-[1-(benzocyclobutén-1-yl méthyl) pipérid-4-yl] N-méthyl carbamate de méthyle, ses isomères optiques et ses sels d'addition avec un acide minéral ou organique pharmaceutiquement acceptable.

**8.** Un composé de la revendication 1 qui est :
Le N-[1-(benzocyclobutén-1-yl méthyl) pipérid-4-yl] N-éthyl carbamate d'éthyle, ses isomères optiques et ses sels d'addition avec un acide minéral ou organique pharmaceutiquement acceptable.

**9.** Un composé de la revendication 1 qui est :
Le N-[1-(benzocyclobutén-1-yl méthyl) pipérid-4-yl] N-méthyl carbamate de phényle, ses isomères optiques et ses sels d'addition avec un acide minéral ou organique pharmaceutiquement acceptable.

**10.** Un composé de la revendication 1 qui est :
Le N-[1-(benzocyclobutén-1-yl méthyl) pipérid-4-yl] N- méthyl carbamate de benzyle, ses isomères optiques et ses sels d'addition avec un acide minéral ou organique pharmaceutiquement acceptable.

**11.** Un composé de la revendication 1 qui est :
Le N-[1-(benzocyclobutén-1-yl méthyl) pipérid-4-yl] N-méthyl carbamate de vinyle, ses isomères optiques et ses sels d'addition avec un acide minéral ou organique pharmaceutiquement acceptable.

**12.** Un composé de la revendication 1 qui est :
Le N-[1-(indan-2-yl) pipérid-4-yl] N-méthyl carbamate d'isobutyle, ses isomères optiques et ses sels d'addition avec un acide minéral ou organique pharmaceutiquement acceptable.

**13.** Un composé de la revendication 1 qui est :
Le N-[1-(indan-2-yl) pipérid-4-yl] N-méthyl carbamate de propyle, ses isomères optiques et ses sels d'addition avec un acide minéral ou organique pharmaceutiquement acceptable.

**14.** Le procédé de préparation des composés de la revendication 1, caractérisé en ce que l'on condense :
- un composé de formule II :

$$R\text{-}(CH_2)_m\text{-}X \qquad (II)$$

dans laquelle R et m ont les significations définies dans la revendication 1, et X représente un atome d'halogène, un radical tosyloxy ou un radical mésyloxy,
- avec un composé de formule III :

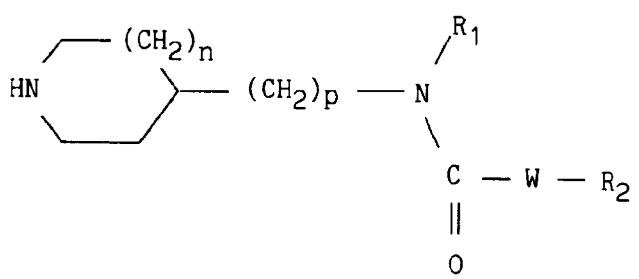

(III)

dans laquelle n, p, $R_1$, W et $R_2$ ont les significations définies dans la revendication 1 ; et si on le désire les composés I ainsi obtenus sont :
- salifiés avec un acide minéral ou organique pharmaceutiquement acceptable,
ou
- séparés en leurs isomères optiques, lesquels peuvent être, à leur tour, salifiés.

**15.** Le procédé de préparation des composés de la revendication 1 , caractérisé en ce que :
- l'on fait réagir un composé de formule IV :

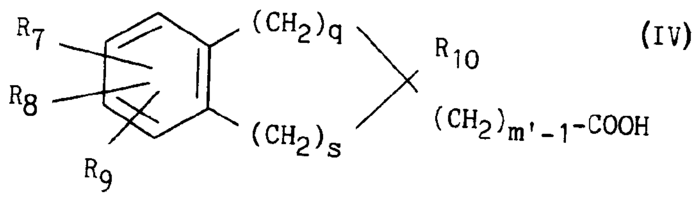

(IV)

dans laquelle $R_7$, $R_8$, $R_9$ et $R_{10}$ ont les significations définies dans la revendication 1, m′ représente 1,2,3 ou 4 et q et s représentent 0,1 ou 2 à condition toutefois que q + s soit égal à 1 ou 2,
avec un composé de formule V :

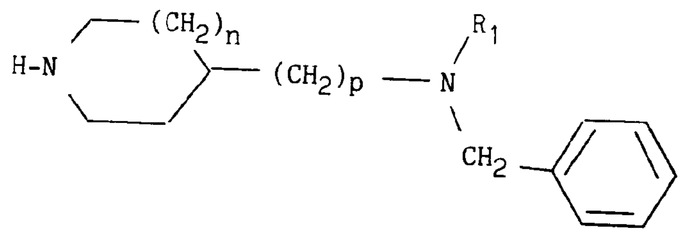

(V)

dans laquelle n, p et $R_1$ ont les significations définies dans la revendication 1, pour former un composé de formule VI :

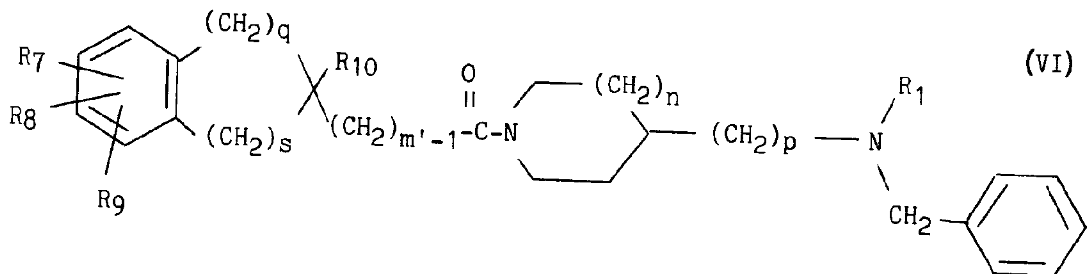

(VI)

dans laquelle $R_1$, $R_7$, $R_8$, $R_9$, $R_{10}$, m′, n, p, q et s ont les significations indiquées ci-dessus,
- que l'on soumet à l'action d'hydrure de lithium et d'aluminium pour former un composé de formule VII :

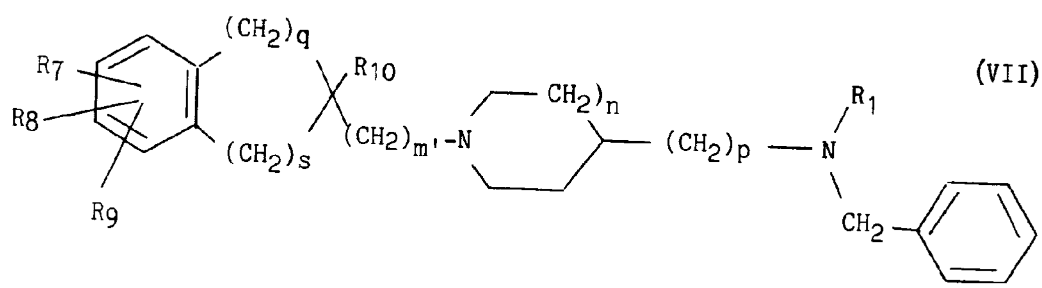

(VII)

dans laquelle la signification de $R_1$, $R_7$, $R_8$, $R_9$, $R_{10}$, m′, n, p, q et s reste identique à celle donnée ci-dessus,
- que l'on soumet à l'action d'hydrogène, en présence d'un catalyseur tel que $PtO_2$ ou Pd/C, dans un solvant alcoolique, en présence d'une quantité stoechiométrique d'acide chlorhydrique ou acétique, pour former un composé de formule VIII :

(VIII)

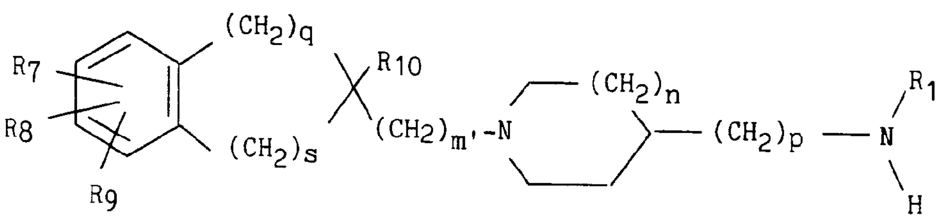

dans laquelle $R_1$, $R_7$, $R_8$, $R_9$, $R_{10}$, m', n , p, q et s ont les significations indiquées ci-dessus, lequel composé VIII est ensuite :

- soit mis à réagir avec un composé de formule IX :

(IX)

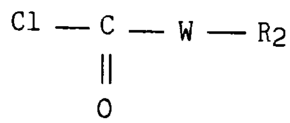

dans laquelle $R_2$ a la signification définie dans la revendication 1 à l'exception d'un atome d'hydrogène, et W représente une simple liaison ou un atome d'oxygène,

pour former les composés de la revendication 1 répondant à la formule I, dans laquelle R représente un radical B ou un radical C, W représente une simple liaison ou un atome d'oxygène, $R_2$ a la signification définie dans la revendication 1 mais ne représente pas un atome d'hydrogène, et m a la signification définie dans la revendication 1 à l'exception de zéro,

- soit mis à réagir avec l'acide formique en présence d'anhydride acétique pour obtenir les composés de la revendication 1 répondant à la formule I dans laquelle R représente un radical B ou un radical C, W représente une simple liaison, $R_2$ un atome d'hydrogène, et m a la signification définie dans la revendication 1, à l'exception de zéro ;

- soit mis à réagir avec un composé de formule X :

$$R_2 - N = C = O \qquad (X)$$

dans laquelle $R_2$ a la même signification que pour la formule I, selon la revendication 1 à l'exception d'un atome d'hydrogène, pour former les composés de la revendication 1 répondant à la formule I dans laquelle R représente un radical B ou un radical C, W représente un radical -NH-, $R_2$ a la signification définie dans la revendication 1 à l'exception d'un atome d'hydrogène, et m a la signification définie dans la revendication 1, à l'exception de zéro ;

et si on le désire les composés I ainsi obtenus sont :

- salifiés avec un acide minéral ou organique pharmaceutiquement acceptable,

ou

- séparés en leurs isomères optiques, lesquels peuvent être, à leur tour, salifiés.

et si on le désire les composés I ainsi obtenus sont :

- salifiés avec un acide minéral ou organique pharmaceutiquement acceptable,

ou

- séparés en leurs isomères optiques, lesquels peuvent être, à leur tour, salifiés.

**16.** Les compositions pharmaceutiques contenant comme principe actif un composé d'une quelconque des revendications 1 à 13, en association ou en mélange avec un excipient ou un véhicule inerte, non toxique, pharmaceutiquement acceptable.

**17.** Les compositions pharmaceutiques selon la revendication 16 renfermant le principe actif à la dose de 0,1 à 100 mg.

**18.** Les compositions pharmaceutiques selon les revendications 16 et 17 renfermant comme principe actif au moins un composé selon les revendications 1 à 13, utilisable dans le traitement de la douleur, du stress, de la migraine, de l'anxiété, de la dépression ou de la schizophrénie.

## Patentansprüche

**1.** Verbindung der Formel I

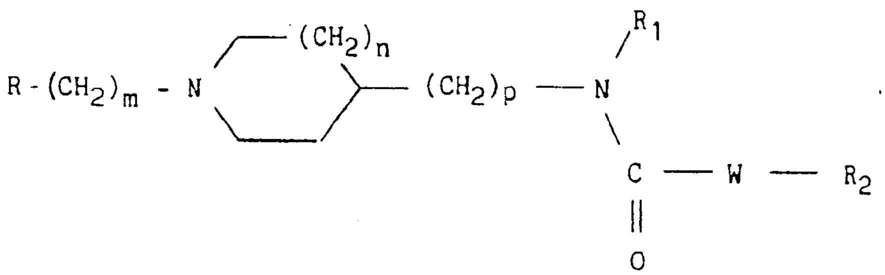

(I)

in der:

- m Null, 1, 2, 3 oder 4,
- n und p Null, 1 oder 2,
- W ein Sauerstoffatom, eine Gruppe -NH- oder eine Einfachbindung,
- R:

eine Benzocyclobuten-1-yl-gruppe der Formel B:

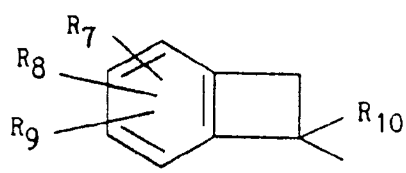

(B)

oder eine Indanylgruppe der Formel C:

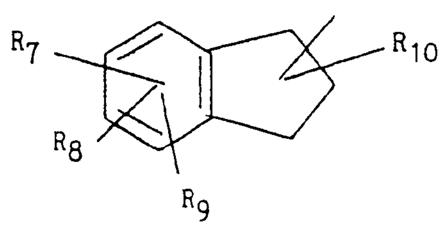

(C)

in denen:

- $R_7$, $R_8$ und $R_9$, die gleichartig oder verschieden sein können, jeweils ein Wasserstoffatom, ein Halogenatom, eine Alkyl- oder Alkoxy-gruppe mit 1 bis 6 Kohlenstoffatomen, eine polyhalogenierte Alkylgruppe mit 1 bis 6 Kohlenstoffatomen, eine Hydroxylgruppe oder $R_7$ und $R_8$ oder $R_8$ und $R_9$ gemeinsam eine Methylendioxygruppe, eine Ethylendioxygruppe, einen Furanring oder einen Dihydrofuranring und
- $R_{10}$ ein Wasserstoffatom oder eine Alkylgruppe mit 1 bis 6 Kohlenstoffatomen darstellen;

- $R_1$ eine Alkylgruppe mit 1 bis 6 Kohlenstoffatomen,
- $R_2$ ein Wasserstoffatom, eine Alkylgruppe mit 1 bis 6 Kohlenstoffatomen, eine Alkengruppe mit 2 bis 6 Kohlenstoffatomen, eine Cycloalkylgruppe mit 4 bis 7 Kohlenstoffatomen, eine Benzylgruppe oder eine Phenylgruppe (die jeweils gegebenenfalls durch ein oder mehrere Halogenatome, Hydroxylgruppen oder Alkyl- oder Alkoxygruppen mit 1 bis 6 Kohlenstoffatomen substituiert sein können), eine Aralkylgruppe mit 7 bis 12 Kohlenstoffatomen, eine Alkoxyalkylgruppe mit 2 bis 7 Kohlenstoffatomen oder eine polyhalogenierte Alkylgruppe mit 1 bis 6 Kohlenstoffatomen bedeuten,

deren optische Isomere und deren Additionssalze mit einer organischen oder anorganischen, pharmazeutisch annehmbaren Säure.

**2.** Verbindung nach Anspruch 1, nämlich:

N-(1-indan-2-yl-piperid-4-yl)-N-methyl-propionamid, dessen optische Isomere und dessen Additionssalze mit einer anorganischen oder organischen, pharmazeutisch annehmbaren Säure.

**3.** Verbindung nach Anspruch 1, nämlich:
N-[1-(Benzocyclobuten-1-yl-methyl)-piperid-4-yl]-N-methyl-carbamidsäurepropylester, dessen optische Isomere und dessen Additionssalze mit einer anorganischen oder organischen, pharmazeutisch annehmbaren Säure.

**4.** Verbindung nach Anspruch 1, nämlich:
N-[1-(Benzocyclobuten-1-yl-methyl)-piperid-4-yl]-N-ethyl-carbamidsäuremethylester, dessen optische Isomere und dessen Additionssalze mit einer anorganischen oder organischen, pharmazeutisch annehmbaren Säure.

**5.** Verbindung nach Anspruch 1, nämlich:
N-[1-(Benzocyclobuten-1-yl-methyl)-piperid-4-yl]-N-ethyl-acetamid, dessen optische Isomere und dessen Additionssalze mit einer anorganischen oder organischen, pharmazeutisch annehmbaren Säure.

**6.** Verbindung nach Anspruch 1, nämlich:
N-[1-(Benzocyclobuten-1-yl-methyl)-piperid-4-yl]-N-methyl-butyramid, dessen optische Isomere und dessen Additionssalze mit einer anorganischen oder organischen, pharmazeutisch annehmbaren Säure.

**7.** Verbindung nach Anspruch 1, nämlich:
N-[1-(Benzocyclobuten-1-yl-methyl)-piperid-4-yl]-N-methyl-carbamidsäureme- thylester, dessen optische Isomere und dessen Additionssalze mit einer anorganischen oder organischen, pharmazeutisch annehmbaren Säure.

**8.** Verbindung nach Anspruch 1, nämlich:
N-[1-(Benzocyclobuten-1-yl-methyl)-piperid-4-yl]-N-ethyl-carbamidsäureethylester, dessen optische Isomere und dessen Additionssalze mit einer anorganischen oder organischen, pharmazeutisch annehmbaren Säure.

**9.** Verbindung nach Anspruch 1, nämlich:
N-[1-(Benzocyclobuten-1-yl-methyl)-piperid-4-yl]-N-methyl-carbamidsäurephenylester, dessen optische Isomere und dessen Additionssalze mit einer anorganischen oder organischen, pharmazeutisch annehmbaren Säure.

**10.** Verbindung nach Anspruch 1, nämlich:
N-[1-(Benzocyclobuten-1-yl-methyl)-piperid-4-yl]-N-methyl-carbamidsäure- benzylester, dessen optische Isomere und dessen Additionssalze mit einer anorganischen oder organischen, pharmazeutisch annehmbaren Säure.

**11.** Verbindung nach Anspruch 1, nämlich:
N-[1-(Benzocyclobuten-1-yl-methyl)-piperid-4-yl]-N-methyl-carbamidsäurevinylester, dessen optische Isomere und dessen Additionssalze mit einer anorganischen oder organischen, pharmazeutisch annehmbaren Säure.

**12.** Verbindung nach Anspruch 1, nämlich:
N-[1-(Indan-2-yl)-piperid-4-yl]-N-methyl-carbamidsäureisobutylester, dessen optische Isomere und dessen Additionssalze mit einer anorganischen oder organischen, pharmazeutisch annehmbaren Säure.

**13.** Verbindung nach Anspruch 1, nämlich:
N-[1-(Indan-2-yl)-piperid-4-yl]-N-methyl-carbamidsäurepropylester, dessen optische Isomere und dessen Additionssalze mit einer anorganischen oder organischen, pharmazeutisch annehmbaren Säure.

**14.** Verfahren zur Herstellung der Verbindungen nach Anspruch 1, **dadurch gekennzeichnet**, daß man
- eine Verbindung der Formel II:

$$R - (CH_2)_m - X \qquad (II)$$

in der R und m die in Anspruch 1 angegebenen Bedeutungen besitzen und X ein Halogenatom, eine Tosyloxygruppe oder eine Mesyloxygruppe bedeutet,
- mit einer Verbindung der Formel III:

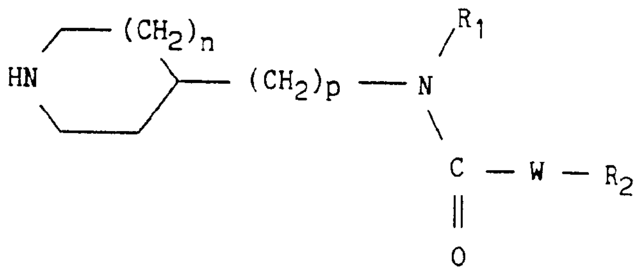

(III)

in der n, p, $R_1$, W und $R_2$ die in Anspruch 1 angegebenen Bedeutungen besitzen, kondensiert; und gewünschtenfalls die in dieser Weise erhaltenen Verbindungen der Formel I:

- mit einer anorganischen oder organischen, pharmazeutisch annehmbaren Säure in ein Salz überführt
oder
- in ihre optischen Isomeren auftrennt, die ihrerseits in Salze überführt werden können.

**15.** Verfahren zur Herstellung der Verbindungen nach Anspruch 1, **dadurch gekennzeichnet**, daß man:

- eine Verbindung der Formel IV:

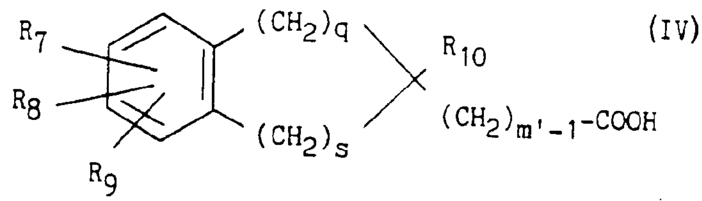

(IV)

in der $R_7$, $R_8$, $R_9$ und $R_{10}$ die in Anspruch 1 angegebenen Bedeutungen besitzen, m' 1, 2, 3 oder 4 und q und s 0, 1 oder 2 mit der Maßgabe bedeuten, daß q + s 1 oder 2 darstellt,
mit einer Verbindung der Formel V:

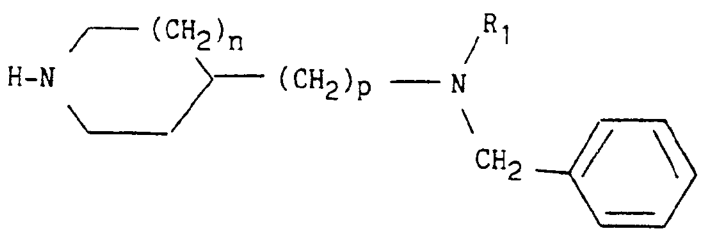

(V)

in der n, p und $R_1$ die in Anspruch 1 angegebenen Bedeutungen besitzen. umsetzt zur Bildung einer Verbindung der Formel VI:

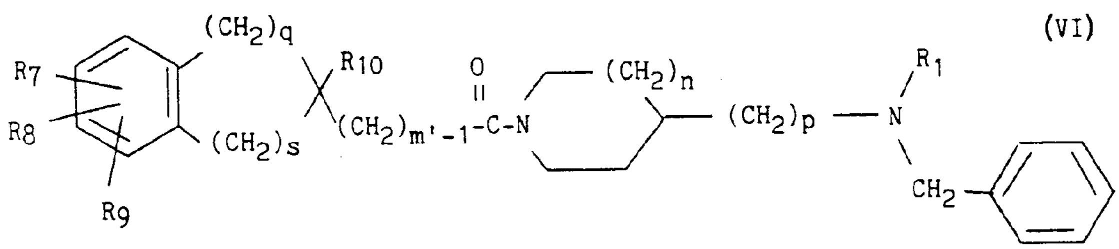

(VI)

in der $R_1$, $R_7$, $R_8$, $R_9$, $R_{10}$, m', n, p, q und s die oben angegebenen Bedeutungen besitzen,

- welche man der Einwirkung von Lithiumaluminiumhydrid unterwirft zur Bildung einer Verbindung der Formel VII:

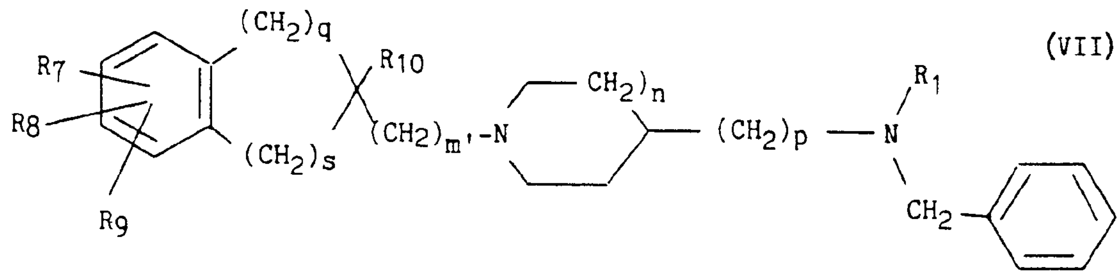

(VII)

in der $R_1$, $R_7$, $R_8$, $R_9$, $R_{10}$, m', n, p, q und s die oben angegebenen Bedeutungen besitzen,

- welche man in Gegenwart eines Katalysators, wie $PtO_2$ oder Pd/C, in einem alkoholischen Lösungsmittel und in Gegenwart einer stöchiometrischen Menge Chlorwasserstoffsäure oder Essigsäure der Einwirkung von Wasserstoff unterwirft zur Bildung einer Verbindung der Formel VIII:

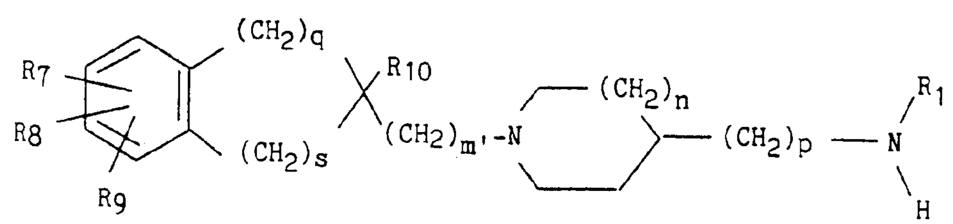

(VIII)

in der $R_1$, $R_7$, $R_8$, $R_9$, $R_{10}$, m', n, p, q und s die oben angegebenen Bedeutungen besitzen, welche Verbindung VIII anschließend:

- entweder mit einer Verbindung der Formel IX:

$$Cl-\underset{\underset{O}{\|}}{C}-W-R_2 \qquad (IX)$$

in der $R_2$ die in Anspruch 1 angegebenen Bedeutungen mit Ausnahme eines Wasserstoffatoms besitzt und W eine Einfachbindung oder ein Sauerstoffatom darstellt, umsetzt, zur Bildung der Verbindungen nach Anspruch 1 der Formel I, in der R eine Gruppe B oder eine Gruppe C darstellt, W eine Einfachbindung oder ein Sauerstoffatom darstellt, $R_2$ die in Anspruch 1 angegebenen Bedeutungen besitzt, jedoch kein Wasserstoffatom darstellt, und m die in Anspruch 1 angegebenen Bedeutungen mit Ausnahme von Null besitzt,

- oder mit Ameisensäure in Gegenwart von Essigsäureanhydrid umsetzt zur Bildung der Verbindungen nach Anspruch 1 der Formel I, in der R eine Gruppe B oder eine Gruppe C darstellt, W eine Einfachbindung bedeutet, $R_2$ ein Wasserstoffatom darstellt und m die in Anspruch 1 angegebenen Bedeutungen besitzt mit Ausnahme von Null;
- oder mit einer Verbindung der Formel X:

$$R_2-N=C=O \qquad (X)$$

in der $R_2$ die bezüglich der Formel I in Anspruch 1 angegebenen Bedeutungen mit Ausnahme eines Wasserstoffatoms besitzt, umsetzt zur Bildung der Verbindungen nach Anspruch 1 der Formel I, in der R eine Gruppe B oder eine Gruppe C darstellt, W eine Gruppe -NH- bedeutet, $R_2$ die in Anspruch 1 angegebenen Bedeutungen besitzt mit Ausnahme des Wasserstoffatoms und m die in Anspruch 1 angegebenen Bedeutungen besitzt mit Ausnahme von Null;

und gewünschtenfalls die in dieser Weise erhaltenen Verbindungen I:

- mit einer anorganischen oder organischen, pharmazeutisch annehmbaren Säure in die Salze überführt,

oder

- in ihre optischen Isomeren auftrennt, die ihrerseits in die Salze überführt werden können.

16. Pharmazeutische Zubereitungen enthaltend als Wirkstoff eine Verbindung nach einem der Ansprüche 1

bis 13 in Kombination oder in Mischung mit einem inerten, nichttoxischen, pharmazeutisch annehmbaren Trägermaterial oder Bindemittel.

**17.** Pharmazeutische Zubereitungen nach Anspruch 16 enthaltend den Wirkstoff in einer Dosis von 0,1 bis 100 mg.

**18.** Pharmazeutische Zubereitungen nach den Ansprüchen 16 und 17 enthaltend als Wirkstoff mindestens eine Verbindung nach den Ansprüchen 1 bis 13, geeignet zur Behandlung von Schmerzen, Streß, Migräne, Angst, Depressionen oder der Schizophrenie.

## Claims

**1.** Compound of formula I

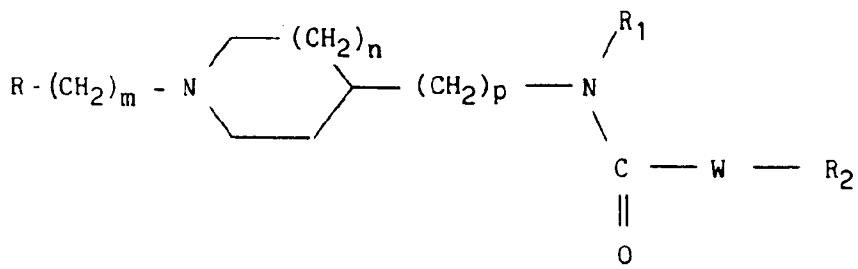

(I)

in which:

- m represents zero, 1, 2, 3 or 4,
- n and p represent zero, 1 or 2,
- W represents an oxygen atom, a radical -NH- or a single bond,
- R represents:

  a benzocyclobuten-1-yl radical of formula B:

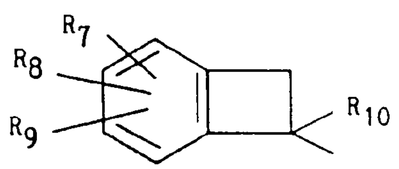

(B)

  or an indanyl radical of formula C

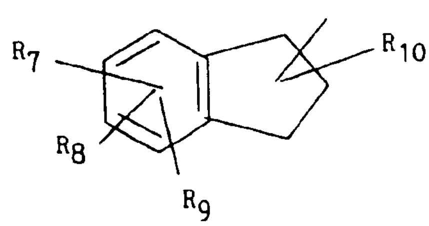

(C)

  in which:
  - $R_7$, $R_8$ and $R_9$, which may be identical or different, each represent a hydrogen atom, a halogen atom, an alkyl or alkoxy radical having from 1 to 6 carbon atoms, a polyhalogenated alkyl radical having from 1 to 6 carbon atoms, or a hydroxy radical, or $R_7$ and $R_8$ or $R_8$ and $R_9$ together form a methylenedioxy radical, an ethylenedioxy radical, a furan ring or a dihydrofuran ring, and
  - $R_{10}$ represents a hydrogen atom or an alkyl radical having from 1 to 6 carbon atoms;
- $R_1$ represents an alkyl radical having from 1 to 6 carbon atoms,
- $R_2$ represents a hydrogen atom, an alkyl radical having from 1 to 6 carbon atoms, an alkenyl radical having from 2 to 6 carbon atoms, a cycloalkyl radical having from 4 to 7 carbon atoms, a benzyl radical

or a phenyl radical (each of which is optionally substituted by one or more halogen atoms, hydroxy radicals, or alkyl or alkoxy radicals having from 1 to 6 carbon atoms), an aralkyl radical having from 7 to 12 carbon atoms, an alkoxyalkyl radical having from 2 to 7 carbon atoms, or a polyhalogenated alkyl radical having from 1 to 6 carbon atoms, their optical isomers and their addition salts with a pharmaceutically acceptable organic or mineral acid.

**2.** A compound according to claim 1 which is:
N-(1-indan-2-ylpiperid-4-yl)-N-methylpropionamide, its optical isomers and its addition salts with a pharmaceutically acceptable mineral or organic acid.

**3.** A compound according to claim 1 which is:
propyl N-[1-(benzocyclobuten-1-ylmethyl)piperid-4-yl]-N-methylcarbamate, its optical isomers and its addition salts with a pharmaceutically acceptable mineral or organic acid.

**4.** A compound according to claim 1 which is:
methyl N-[1-(benzocyclobuten-1-ylmethyl)piperid-4-yl]-N-ethylcarbamate, its optical isomers and its addition salts with a pharmaceutically acceptable mineral or organic acid.

**5.** A compound according to claim 1 which is:
N-[1-(benzocyclobuten-1-ylmethyl)piperid-4-yl]-N-ethylacetamide, its optical isomers and its addition salts with a pharmaceutically acceptable mineral or organic acid.

**6.** A compound according to claim 1 which is:
N-[1-(benzocyclobuten-1-ylmethyl)piperid-4-yl]-N-methylbutyramide, its optical isomers and its addition salts with a pharmaceutically acceptable mineral or organic acid.

**7.** A compound according to claim 1 which is:
methyl N-[1-(benzocyclobuten-1-ylmethyl)piperid-4-yl]-N-methylcarbamate, its optical isomers and its addition salts with a pharmaceutically acceptable mineral or organic acid.

**8.** A compound according to claim 1 which is:
ethyl N-[1-(benzocyclobuten-1-ylmethyl)piperid-4-yl]-N-ethylcarbamate, its optical isomers and its addition salts with a pharmaceutically acceptable mineral or organic acid.

**9.** A compound according to claim 1 which is:
phenyl N-[1-(benzocyclobuten-1-ylmethyl)piperid-4-yl]-N-methylcarbamate, its optical isomers and its addition salts with a pharmaceutically acceptable mineral or organic acid.

**10.** A compound according to claim 1 which is:
benzyl N-[1-(benzocyclobuten-1-ylmethyl)piperid-4-yl]-N-methylcarbamate, its optical isomers and its addition salts with a pharmaceutically acceptable mineral or organic acid.

**11.** A compound according to claim 1 which is:
vinyl N-[1-(benzocyclobuten-1-ylmethyl)piperid-4-yl]-N-methylcarbamate, its optical isomers and its addition salts with a pharmaceutically acceptable mineral or organic acid.

**12.** A compound according to claim 1 which is:
isobutyl N-[1-(indan-2-yl)piperid-4-yl]-N-methylcarbamate, its optical isomers and its addition salts with a pharmaceutically acceptable mineral or organic acid.

**13.** A compound according to claim 1 which is:
propyl N-[1-(indan-2-yl)piperid-4-yl]-N-methylcarbamate, its optical isomers and its addition salts with a pharmaceutically acceptable mineral or organic acid.

**14.** Process for the preparation of a compound according to claim 1, characterised in that:

- a compound of formula II:

$$R\text{-}(CH_2)_m\text{-}X \qquad (II),$$

in which R and m have the meanings defined in claim 1 and X represents a halogen atom, a tosyloxy radical or a mesyloxy radical, is condensed

- with a compound of formula III:

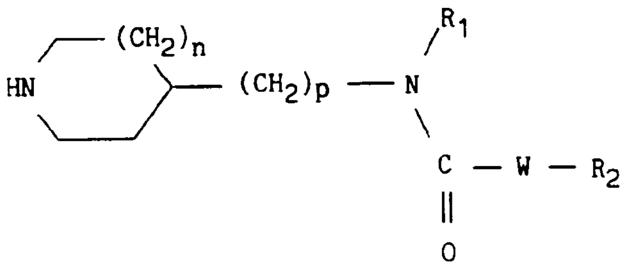

(III),

in which n, p, $R_1$, W and $R_2$ have the meanings defined in claim 1; and, if desired, the compounds I so obtained are:

- converted into salts with a pharmaceutically acceptable mineral or organic acid,
  or
- separated into their optical isomers, which may, in turn, be converted into salts.

**15.** Process for the preparation of a compound according to claim 1, characterised in that:

- a compound of formula IV:

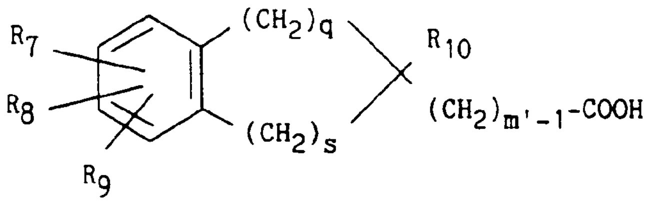

(IV),

in which $R_7$, $R_8$, $R_9$ and $R_{10}$ have the meanings defined in claim 1, m′ represents 1, 2, 3 or 4 and q and s represent 0, 1 or 2, with the proviso that q + s is equal to 1 or 2,
is reacted with a compound of formula V:

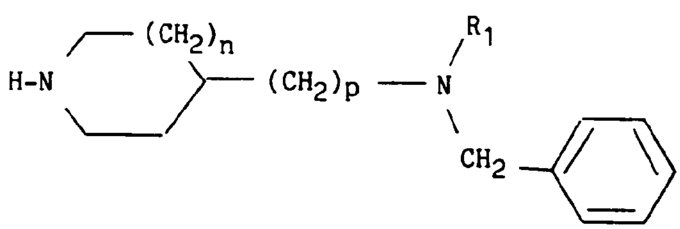

(V),

in which n, p and $R_1$ have the meanings defined in claim 1, to form a compound of formula VI:

(VI)

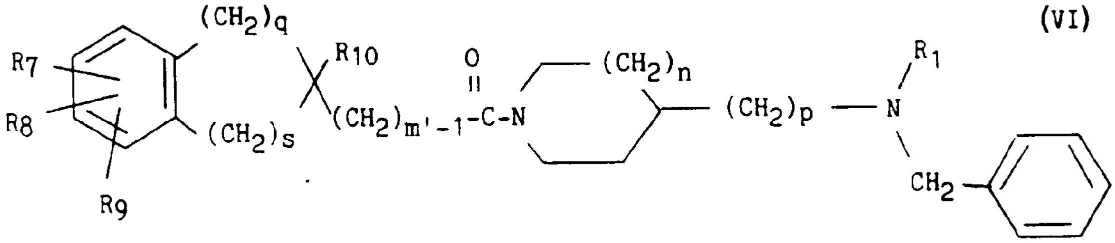

in which $R_1$, $R_7$, $R_8$, $R_9$, $R_{10}$, m′, n, p, q and s have the meanings indicated above,

- which is subjected to the action of lithium aluminium hydride to form a compound of formula VII:

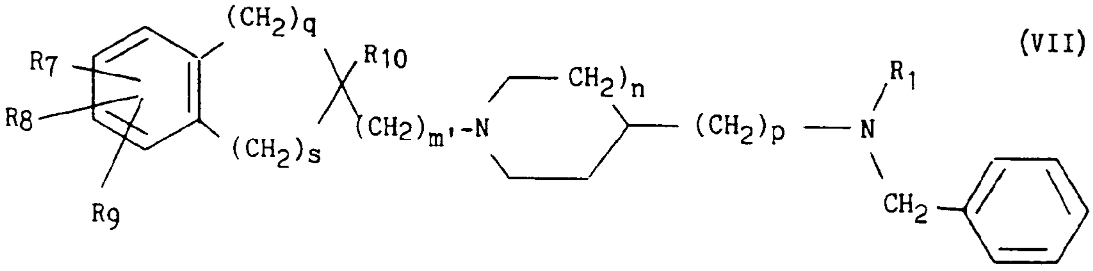

(VII)

in which the meaning of $R_1$, $R_7$, $R_8$, $R_9$, $R_{10}$, m′, n, p, q and s is identical to that given above,

- which is subjected to the action of hydrogen, in the presence of a catalyst such as $PtO_2$ or Pd/C, in an alcoholic solvent, in the presence of a stoichiometric amount of hydrochloric acid or acetic acid, to form a compound of formula VIII:

(VIII)

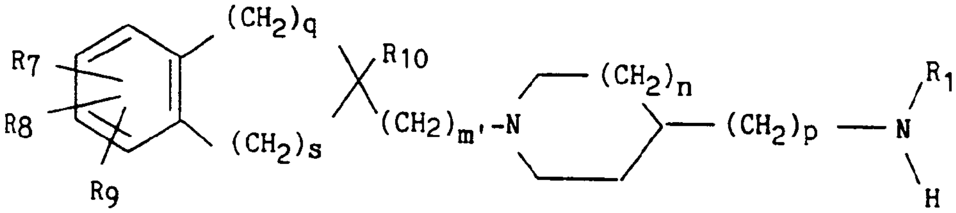

in which $R_1$, $R_7$, $R_8$, $R_9$, $R_{10}$, m′, n, p, q and s have the meanings indicated above, which compound VIII is then:

- reacted with a compound of formula IX:

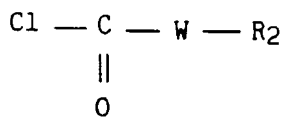

(IX),

in which $R_2$ has the meaning defined in claim 1 with the exception of a hydrogen atom, and W represents a single bond or an oxygen atom,
to form the compounds according to claim 1 corresponding to formula I in which R represents a radical B or a radical C, W represents a single bond or an oxygen atom, $R_2$ has the meaning defined in claim 1 but does not represent a hydrogen atom, and m has the meaning defined in claim 1 with the exception of zero,
- or reacted with formic acid in the presence of acetic anhydride to give the compounds according to claim 1 corresponding to formula I in which R represents a radical B or a radical C, W represents a single bond, $R_2$ represents a hydrogen atom, and m has the meaning defined in claim 1 with the exception of zero;
- or reacted with a compound of formula X:

$$R_2\text{-N=C=O} \qquad (X),$$

in which $R_2$ has the same meaning as for formula I according to claim 1 with the exception of a hydrogen atom, to form the compounds according to claim 1 corresponding to formula I in which R represents a radical B or a radical C, W represents a radical -NH-, $R_2$ has the meaning defined in claim 1 with the exception of a hydrogen atom, and m has the meaning defined in claim 1 with the exception of zero;
and, if desired, the compounds I so obtained are:
- converted into salts with a pharmaceutically acceptable mineral or organic acid,
or
- separated into their optical isomers, which may, in turn, be converted into salts.

**16.** Pharmaceutical compositions containing as active ingredient a compound according to any one of claims 1 to 13, in association or in admixture with a pharmaceutically acceptable, inert, non-toxic excipient or carrier.

**17.** Pharmaceutical compositions according to claim 16 containing the active ingredient in an amount of from 0.1 to 100 mg.

**18.** Pharmaceutical compositions according to claims 16 and 17 containing as active ingredient at least one compound according to claims 1 to 13, for use in the treatment of pain, stress, migraine, anxiety, depression or schizophrenia.